# EUROPEAN PATENT APPLICATION

(11) **EP 4 835 413 A1**
(43) Date of publication of application: **04.11.2026**
(21) Application number: 25173084.2
(22) Date of filing: 29.04.2025
(51) Int. Cl.: G01G 3/16, G01K 11/00, G01N 29/24

(54) **A SENSOR DEVICE AND A METHOD FOR ACQUIRING INFORMATION OF A MEASURAND**

(71) Applicant: Imec VZW, 3001 Leuven (BE); Katholieke Universiteit Leuven KU Leuven Research & Development, 3000 Leuven (BE)
(72) Inventor: LAMBERTI, Fabrice-Roland, 00138 Rome (IT); PIETERS, Cedric, 2880 Bornem (BE); KJELLMAN, Jon, 2800 Kongens Lyngby (DK); KEULEMANS, Grim, 1030 Schaarbeek (BE); BROUCKAERT, Joost, 8500 Kortrijk (BE); JANSEN, Roelof, 3001 Heverlee (BE); ROTTENBERG, Xavier, 3010 Kessel-Lo (BE)
(74) Representative: AWA Sweden AB

(57) **Abstract**

A sensor device (100) comprises: a plurality of sensors (110) each configured to be affected by a measurand; and a waveguide (120) associated with a group of sensors (112) and configured to receive an optical interrogation signal comprising a plurality of separate interrogation wavelengths; wherein the sensor device (100) is configured to, for each sensor (110), individually control a relation between the plurality of separate interrogation wavelengths and a resonance wavelength of optical resonance of the sensor (110); wherein the sensor device (100) further comprises a detector (140) configured to detect a measurement signal representing a linear combination of individual measurements of the measurand by the sensors in the group of sensors (112); wherein the sensor device (100) is configured to perform a series of measurements of the measurand for detecting measurement signals representing different linear combinations of the individual measurements of the measurand by the sensors.

## Description

### Technical field

The present description relates to a sensor device comprising a plurality of sensors, each comprising an optical resonance element. The optical resonance element is configured to be affected by a measurand for detecting the measurand. The present description also relates to a method for acquiring information of the measurand.

### Background

Optical resonance elements are used in various sensor applications. The optical resonance elements define an optical resonance at an optical wavelength. The optical resonance may be modified by a measurand that is to be measured such that the measurand may be determined based on an effect on optical resonance caused by the measurand.

The optical resonance elements may for instance be used in photo-acoustic imaging, wherein a measurand in form of an acoustic wave may be detected based on the acoustic wave affecting optical resonance of an optical resonance element. The acoustic wave may for instance provide a response from living tissue being subject to an optical pulse which generates the acoustic wave in the tissue. In order for an image to be formed based on detection of the acoustic wave, a large number of optical resonance elements, each associated with a respective sensor, may be needed.

In view of using a plurality of sensors having optical resonance elements, there is a need for an efficient manner of reading signals from the plurality of sensors. In particular, there is a need to allow efficient reading of signals from the plurality of sensors with a high signal-to-noise ratio.

### Summary

An objective of the present description is to provide control of readout of optical signals providing information of detection of a measurand for efficiently reading out the optical signals. A particular objective is to provide readout of optical signals with a high signal-to-noise ratio (SNR).

This and other objectives are at least partly met by the invention as defined in the independent claims. Preferred embodiments are set out in the dependent claims.

According to a first aspect, there is provided a sensor device comprising: a plurality of sensors, wherein each sensor comprises an optical resonance element, wherein an optical characteristic of the optical resonance element is configured to be affected by a measurand to be measured by the sensor; and a waveguide associated with a group of sensors among the plurality of sensors, wherein the waveguide is configured to receive an optical interrogation signal comprising a plurality of separate interrogation wavelengths; wherein the sensor device is configured to, for each sensor, individually control a relation between the plurality of separate interrogation wavelengths and a resonance wavelength of optical resonance of the sensor; wherein the sensor device further comprises a detector configured to receive an optical readout signal, wherein the optical readout signal is based on interaction of the optical interrogation signal with each of the sensors in the group of sensors, wherein the detector is configured to detect a measurement signal based on an intensity of the optical readout signal, wherein the measurement signal represents a linear combination of individual measurements of the measurand by the sensors in the group of sensors; wherein the sensor device is configured to perform a series of measurements of the measurand, wherein the sensor device is configured to provide different settings of the relations between the plurality of separate interrogation wavelengths and the resonance wavelengths of the sensors for different measurements in the series, wherein the detector is configured to detect a series of measurement signals for the series of measurements, wherein the measurement signals in the series of measurements signals represent different linear combinations of the individual measurements of the measurand by the sensors in the group of sensors.

The sensor device is configured to use multiple interrogation wavelengths and a series of measurements of the measurand. Thus, the sensor device uses a combination of wavelength multiplexing and time multiplexing for enabling determining information of the measurand acquired by each of the sensors of the plurality of sensors.

The sensor device is configured to control the relations between interrogation wavelengths and resonance wavelengths of the sensors for forming linear combinations of individual measurements of the measurand by the sensors, wherein the linear combination is detected by detecting a measurement signal. By providing different settings, different linear combinations are provided for different measurement signals in a series. This enables the individual measurements to be extracted from the series of measurement signals.

Thanks to using a linear combination of the individual measurements, the measurement signal may be detected by a single light-sensitive element. There is no need for splitting the optical readout signal for separately detecting the different interrogation wavelengths. Rather, a combined intensity of the interrogation wavelengths may be detected by the detector. This implies that a simple set-up may be used. For instance, a single detector per waveguide may be used.

Further, the use of the linear combination of the individual measurements in the series of measurement signals may allow the individual measurements to be extracted with a high SNR. In particular, the linear combinations may be selected such that an SNR of the individual measurements may be higher than a corresponding SNR based on reading out each sensor individually, one at a time.

The sensor device may be used for sensing a measurand that may be provided in repeated manner. Thus, the sensor device may be configured to detect a response from an object, e.g., for detecting an acoustic signal provided by the object in response to an excitation signal, wherein the object may be static and the series of measurements may be performed for a series of pulses of the excitation signal generating responses from the object. Since the measurement may be repeated, contribution of the individual measurements from different sensors may be accurately extracted from the series of measurement signals.

Within a measurement signal, each sensor contributing to the measurement signal may be uniquely associated with one interrogation wavelength of the plurality of separate interrogation wavelengths. Thus, each of the interrogation wavelengths carry information related to a single individual measurement, respectively.

A number of separate interrogation wavelengths may correspond to a number of sensors within a group of sensors. This implies that all of the individual measurements by the sensors in the group may contribute, at least for some settings, to the linear combination in the measurement signal. However, it should be realized that in some settings, not all of the sensors may be activated to contribute to the linear combination.

It should also be realized that the number of separate interrogation wavelengths may be smaller than the number of sensors within a group of sensors. This implies that the series of measurement signals may comprise two or more sub-series of measurement signals, each sub-series being based on individual measurements by a sub-group of sensors within the group of sensors. Thus, a further time-multiplexing may be used for determining individual measurements by a large number of sensors, such that the number of separate interrogation wavelengths need not match the number of sensors in the group of sensors.

The sensor device may thus enable a waveguide to be associated with a group of sensors that includes a large number of sensors. The sensors within the group may then be controlled in a time-multiplexed manner for readout of individual measurements by all of the sensors in the group. Thus, even though the measurand may affect a non-selected sensor during a readout time point, the non-selected sensor may be controlled not have a resonance wavelength interfering with the interrogation wavelengths such that the non-selected sensor will not affect the linear combination of individual measurements of selected sensors in the group.

As used herein, the term "*measurand*" should be construed as any quantity, object, or property intended to be measured. Given as a non-limiting example, the measurand may be a temperature, a mass, or another quantity. Given as another non-limiting example, the measurand may be electro-magnetic radiation or an acoustic signal incident on the optical resonance element. The measurand may be representative of an object interacting with or emitting the electro-magnetic radiation or the acoustic signal. Given as yet another non-limiting example, the measurand may be an object that interacts with the optical resonance element.

The optical resonance element may be affected by the measurand in a manner that may be read out using the optical readout signal based on the optical interrogation signal, such that a measurement of the measurand is acquired by the sensor.

The optical resonance element may be affected by the measurand such that any optical characteristic is affected. For instance, the optical resonance is affected, e.g., the measurand may affect the optical resonance element such that a resonance wavelength may be slightly changed. However, the optical resonance element may alternatively or additionally be affected by the measurand such that a transmission, reflection, absorption, or scattering by the optical resonance element may be directly affected.

The optical resonance element defines optical resonances. The optical resonance element may be designed in many different ways for defining the optical resonances. For instance, the optical resonance element may comprise a loop, such as a ring, disk, or sphere, for allowing an optical signal to travel around the loop. At a resonance wavelength, constructive interference occurs for optical signals making different number of round-trips around the loop. Alternatively, the optical resonance element may comprise a Fabry-Pérot resonator, defining an optical resonator between two reflective elements. As yet another alternative, the optical resonance element may comprise a photonic crystal structure.

According to an embodiment, the optical resonance element is a waveguide resonator. Thus, the optical resonance element may provide a well-defined optical path in a waveguide.

It should be realized that each optical resonance element has a set of resonance wavelengths. The control of the relation between interrogation wavelengths and the resonance wavelength may provide control of one of the resonance wavelengths of the sensor to be aligned with one of the interrogation wavelengths.

The optical interrogation signal may be considered as having a plurality of signal components, wherein each signal component has a unique interrogation wavelength, whereby the plurality signal components have the plurality of separate interrogation wavelengths. The optical interrogation signal may be further configured to interact with the optical resonance element. Interaction of the optical interrogation signal with the optical resonance element may be limited to the signal component having an interrogation wavelength within a bandwidth of an optical mode of the optical resonance element. Hence, only one of the separate interrogation wavelengths with which the resonance wavelength of the optical resonance element of a sensor is aligned may interact with the sensor.

For instance, the optical interrogation signal may be coupled into the optical resonance element in dependence of the interrogation wavelength. Hence, transmission of the signal component by the optical resonance element may depend on how well the interrogation wavelength of the signal component corresponds to the resonance wavelength of the sensor. Signal components having interrogation wavelengths that are not related to the resonance wavelength of a sensor, i.e., interrogation wavelengths that are not spectrally located in the optical mode of the sensor, will not be affected by a measurand affecting the optical resonance element. Such signal components may possibly be affected by being attenuated by the optical resonance element, but the signal components will not be affected by the measurand.

According to an embodiment, a signal component with an interrogation wavelength being aligned with the resonance wavelength may be coupled into the optical resonance element of the sensor such that intensity of the optical interrogation signal is reduced at the signal component. However, it should be realized that, in other embodiments, the optical resonance element may be configured to provide a maximum transmission at the resonance wavelength such that intensity of the optical interrogation signal transmitted by the optical resonance element is high if the interrogation wavelength of the signal component matches well with the resonance wavelength.

Each sensor may be configured to sense the measurand by the optical resonance wavelength of the optical resonance element being changed in dependence of the measurand. The resonance wavelength of the optical resonance element may thus be slightly changed or modulated by the measurand. This implies that interaction of a signal component, having the interrogation wavelength aligned with the resonance wavelength of the optical resonance element, and the optical resonance element may be altered by the measurand. For instance, the interaction may be provided by coupling of the signal component of the optical interrogation signal into the optical resonance element. In this manner, transmission, or alternatively reflection, absorption, or scattering, of the signal component of the optical interrogation signal by the optical resonance element, may be altered by the measurand. This implies that the optical intensity of the signal component of the optical interrogation signal with which the optical resonance element is aligned will be modulated by the measurand.

The optical resonance wavelength of the optical resonance element may define one characteristic of an optical mode. The signal component of the optical interrogation signal may be coupled to the optical mode of the optical resonance element even if the interrogation wavelength of the signal component does not exactly match the optical resonance wavelength. Thus, even if the optical resonance wavelength is modulated by the measurand, the interrogation wavelength of the signal component of the optical interrogation signal with which the optical resonance element is aligned may still be coupled to the optical mode. However, the interaction between the signal component and the optical resonance element may be affected by the modulation of the resonance wavelength caused by the measurand, such that transmission, reflection, absorption, or scattering, of the optical interrogation signal by the optical resonance element may be affected. Also, or alternatively, the interaction between the signal component with the interrogation wavelength and the optical resonance element may be affected by the measurand, such that transmission, reflection, absorption, or scattering of the optical interrogation signal by the optical resonance element may be directly affected by the measurand.

The signal component of the optical interrogation signal may be coupled into the optical resonance element, which may be used for causing transmission, reflection, absorption, or scattering of the signal component. However, the signal component may interact with the optical resonance element in alternative manners, such as to cause a phase change of the signal component.

The optical resonance element may be configured in many different manners such that the measurand alters the resonance wavelength or directly alters transmission, reflection, absorption, or scattering of the signal component with the interrogation wavelength of the optical interrogation signal by the optical resonance element.

For instance, the optical resonance element may comprise an optical waveguide forming a loop, which is arranged on a membrane. Alternatively, the optical resonance element is placed in vicinity of the membrane so that it is optically and/or mechanically coupled to the membrane. Thus, a measurand may affect the membrane so as to alter the shape of the membrane such that a length of the loop of the optical waveguide is modulated so as to modulate the optical resonance wavelength. Alternatively, or additionally, an optical property, such as the refractive index of the optical resonance element or of a material surrounding the optical resonance element may be modulated so as to modulate the optical resonance wavelength. An optical resonator coupled to a mechanical system defines an opto-mechanical system.

According to an alternative embodiment, the measurand may affect coupling of the optical interrogation signal into an optical waveguide of the optical resonance element or the measurand may affect loss of the optical interrogation signal in the optical waveguide. Thus, the measurand may alter transmission of the signal component with the interrogation wavelength of the optical interrogation signal by the optical resonance element.

The sensor may be configured for the optical resonance element being directly affected by the measurand. For instance, the measurand may be configured to directly affect the resonance wavelength of the optical resonance element. This may for instance be provided by the measurand being an optical signal or a substance affecting the optical resonance element.

However, the sensor may alternatively be configured for the optical resonance element being indirectly affected by the measurand. For instance, the optical resonance element may be arranged on a membrane which may be deflected in response to the measurand, whereby a deflection of the membrane may affect the resonance wavelength of the optical resonance element. This may for instance be provided by the measurand being an acoustical signal incident on the membrane or a mass landing on the membrane affecting the optical resonance element arranged on the membrane.

Thus, it should be realized that the performing of a series of measurements of measurands using sensors for which relations between interrogation wavelengths and resonance wavelengths are controlled may be used for various types of sensors.

It should be further realized that the sensor may be adapted to detecting a particular frequency. For instance, the sensor may comprise a membrane having a resonance frequency such that the membrane may be particularly sensitive to a measurand exhibiting a corresponding frequency. It should be realized that the optical resonance element may be arranged on the membrane such that vibrations of the membrane may affect the optical resonance element to allow the measurand to affect the optical characteristics of the optical resonance element. However, the optical resonance element may be provided with a resonance wavelength independently of the resonance frequency of the membrane. Thus, the resonance wavelength of the optical resonance element is not necessarily tied to wavelength or frequency information of the measurand that is to be acquired by the sensor device.

The sensor device comprises a plurality of sensors. It should be realized that the sensors may be arranged in a regular manner forming an array of sensors. The plurality of sensors may be arranged in a one-dimensional array being arranged along a line or in a two-dimensional array being arranged in rows and columns. However, it should be realized that the sensors need not necessarily be arranged with a particular spatial relation between the sensors.

It should be realized that the sensor device may comprise a large number of sensors. Since the sensor device allows for readout of measurements in a systematic manner, the sensor device is particularly suited for use with a large number of sensors. However, it should be realized that the sensor device need not necessarily include a large number of sensors.

It should be realized that the sensor device may comprise a plurality of waveguides, such that each waveguide is associated with a distinct group of sensors. Thus, the sensors may be divided into a number of different groups. However, according to an alternative, the sensor device may comprise a single waveguide. In such case, the waveguide may be associated with a group of sensors corresponding to all of the sensors of the plurality of sensors.

The waveguide may be any structure providing guiding of an optical signal. The waveguide may be configured to confine light in a cross-section perpendicular to an extension of the waveguide. The waveguide may thus be configured to restrict light to follow a path defined by the extension of the waveguide, whereby transmission of light with low loss may be provided. The waveguide may for instance guide light by light being reflected on inner walls of the waveguide by total internal reflection or by a reflective coating being provided on the walls of the waveguide. The waveguide is configured to propagate the optical interrogation signal along the path of the waveguide.

The waveguide may be configured to extend in a path passing all sensors in the group of sensors. The waveguide may be configured to pass at least in vicinity of the optical resonance elements of the sensors in the group of sensors. The optical interrogation signal may thus be coupled into the optical resonance element and this coupling of the optical interrogation signal into the optical resonance element may be used for information of measurement to be carried by the optical interrogation signal.

The optical interrogation signal comprises a plurality of separate interrogation wavelengths. The optical interrogation signal thus comprises a plurality of signal components, wherein each signal components has a unique interrogation wavelength. The optical interrogation signal may thus carry information of measurements from a plurality of sensors by the different signal components. Each signal component of the optical interrogation signal may carry information from measurements from a single sensor. Thanks to the signal components having different interrogation wavelengths, each signal component may be associated with a single sensor, enabling differentiating the information from different sensors form the series of measurements.

The interrogation wavelengths may be separate defining distinct spectral lines of the optical interrogation signal. The interrogation wavelengths may be spectrally separated by a wavelength difference being larger than a width of a resonance curve around the resonance wavelength of the optical resonance elements. This implies that a single interrogation wavelength may interact with the optical resonance element of a single sensor, such that the single sensor will at least not significantly affect more than one signal component having different interrogation wavelengths. For instance, the interrogation wavelengths may be separated in wavelength by a difference corresponding to at least a full width half maximum (FWHM) of the resonance curve of the optical resonance element or a few times the FWHM, such as three times the FWHM.

Each sensor may be configured to modulate the optical intensity at the interrogation wavelength with which the resonance wavelength is aligned. The sensor may modulate the optical intensity by the sensor being affected by the measurand.

The optical interrogation signal may be formed by laser light comprising signal components at the plurality of separate interrogation wavelengths. However, it should be realized that the optical interrogation signal may in some embodiments not necessarily be formed by laser light, but rather non-coherent light may be used. This may be used for instance with optical sensors, wherein a high sensitivity of the optical sensor is not needed.

The sensor device is configured to control the relation between the interrogation wavelengths and the resonance wavelengths of the sensors. This may be performed by tuning the interrogation wavelengths and/or by tuning the resonance wavelengths. For each measurement in a series of measurements, each interrogation wavelength may be uniquely associated with a single sensor. Thus, for each measurement, each interrogation wavelength may be assigned to a unique sensor. The sensor device may then be configured, for each measurement, to control the relation between the interrogation wavelength, associated with the sensor during the measurement, and the resonance wavelength of the sensor with which the interrogation wavelength is associated for providing different settings of the relation for different measurements in the series of measurements. This implies that the contribution to the linear combination of the individual measurements is changed.

The controlling of the relation may thus control an effect of a measurand on the signal component at the interrogation wavelength. Thus, the relation may define a factor applied to the effect of the measurand, as sensed by the sensor, on the signal component at the interrogation wavelength. This provides a factor for the linear combination of the individual measurement by the sensor contributing to the measurement signal.

The sensor device is configured to provide individual control of the relations between the interrogation wavelengths and the resonance wavelength. Thus, each sensor is controlled individually such that the factors for the linear combination of the individual measurements may be separately controlled. This implies that different settings for different measurements may be easily provided.

An interrogation wavelength may be associated with a unique sensor. Thus, for each sensor, the controlling of the relation may imply that the relation is controlled between the interrogation wavelength associated with the sensor and the resonance wavelength of the sensor.

The controlling of the relation between the interrogation wavelengths and the resonance wavelength of a sensor may, for some settings, imply that no interrogation wavelength is matched with the resonance wavelength. Thus, in such setting, the contribution of the sensor to the linear combination is defined by a factor 0. The controlling of the relation to define the factor 0 for the linear combination may be achieved by tuning the interrogation wavelength associated with the sensor, or by controlling the resonance wavelength of the sensor, such that the interrogation wavelength is outside a resonance curve of the sensor. However, the controlling of the relation to define the factor 0 may alternatively be achieved by turning off the signal component at the interrogation wavelength associated with the sensor, during the measurement.

Controlling the relation between the interrogation wavelength and the resonance wavelength for aligning the resonance wavelength with the interrogation wavelength implies that the interrogation wavelength falls within a resonance curve associated with the resonance wavelength. It should be understood that the resonance wavelength is associated with a resonance curve, such that the optical resonance element may interact with the interrogation wavelength even if the peak wavelength of the resonance curve does not exactly match the interrogation wavelength. Thus, aligning the resonance wavelength with the interrogation wavelength does not imply that the peak resonance wavelength is exactly matched to the interrogation wavelength. Rather, an effect of the measurand on the optical resonance element may even be easier to read out if the resonance wavelength or the interrogation wavelength is tuned such that the interrogation wavelength is arranged at a flank of the resonance curve.

The sensor device may be associated with a detector for detecting an optical readout signal. For instance, the detector may detect the optical readout signal, which is formed by the optical interrogation signal after the optical interrogation signal has interacted with the sensors. The detector may be configured to detect an intensity of incident light on the detector. For instance, the detector may be used for detecting intensity of light of the optical readout signal. The intensity of incident light may provide a representation of modulation of the optical readout signal by the measurand in each of the individual measurements by the sensors. Thus, the intensity of incident light is based on a combination of the individual measurements, each contributing to the intensity of a signal component at a respective interrogation wavelength.

The detector may be configured to detect a combined intensity of the signal components at different interrogation wavelengths of the optical readout signal. Thus, the detector may be configured to detect the linear combination of the individual measurements, and the individual measurements may be extracted from the series of measurement signals.

The detector may be configured to receive a transmitted portion of the optical interrogation signal forming the optical readout signal. If an optical resonance translates into a minimum of transmission and the interrogation wavelength of the signal component corresponds well with the optical resonance wavelength, an intensity of the signal component of the optical readout signal reaching the detector is therefore low. If an optical resonance translates into a maximum of transmission and the interrogation wavelength of the signal component corresponds well with the optical resonance wavelength, an intensity of the signal component of the optical readout signal reaching the detector is therefore high. The detector may alternatively be configured to receive a reflected, absorbed, or scattered portion of the optical interrogation signal forming the optical readout signal. Again, if an optical resonance translates into a maximum/minimum of reflection, absorption, or scattering, and if the interrogation wavelength of the signal component corresponds well with the optical resonance wavelength, an intensity of the signal component of the optical readout signal is therefore high/low, respectively.

The detector may be connected to the waveguide for receiving light having propagated through the waveguide past each of the sensors in the group of sensors. Thus, the optical readout signal may be formed by the optical interrogation signal having interacted with the sensors. However, according to an alternative, a portion of the optical interrogation signal may be coupled from the waveguide into the optical resonance element in each sensor based on the interaction between the optical interrogation signal and the optical resonance element, and the portion of the optical interrogation signal may further be coupled from the optical resonance element into a readout waveguide. Thus, an optical readout signal may be formed in the readout waveguide based on coupling of parts of the optical interrogation signal into optical resonance elements. The readout waveguide may further be connected to the detector for propagating the optical readout signal to the detector.

The detector may be configured in any manner for detecting light incident on a light-sensitive area of the detector. For instance, the detector may be configured to convert an intensity of incident light to an electrical signal representing the intensity. The detector may for instance be implemented as a complementary metal-oxide-semiconductor (CMOS sensor), a charge-coupled device, a photodiode, or a photo-multiplier tube.

The optical readout signal may comprise a plurality of signal components at the interrogation wavelengths. The signal components may each represent an individual measurement by a respective sensor. The signal components may further provide separate contributions to the intensity of the optical readout signal as read out by the detector. A wavelength difference between different signal components may be large enough such that a beating signal caused by this difference is too fast to be measured by the detector, since the beating frequency is larger than the detector bandwidth. Hence, the detector measures the coherent sum of the individual measurements of the sensors, while rejecting the beating signals.

Thus, the effect of the measurand on each sensor is represented in combination in the optical readout signal comprising the plurality of signal components. The individual measurements are linearly combined in the optical readout signal. In other words, the optical readout signal may represent a sum of the individual measurements, wherein each individual measurement is associated with a respective factor. The factor for each individual measurement in the linear combination may be set by controlling the relation between the interrogation wavelength and the resonance wavelength of the sensor. Thus, the settings of the relations in a measurement may define factors for the individual measurements in the linear combination detected by the detector.

The sensor is configured to perform a series of measurements. This implies that the sensor is configured to perform a plurality of measurements, one after another in time, to form the series of measurements. Each measurement may be performed with corresponding conditions, such that the measurand to be measured may have same or substantially the same characteristics during each measurement. This implies that the information relating to the measurand acquired form the series of measurements may be processed for extracting the individual measurements from the measurement signals.

Each measurement in the series may have unique settings. In other words, a set of relations between the plurality of interrogation wavelengths and the resonance wavelengths for the sensors in the group of sensors may be unique for each measurement. Thus, the relation for at least one of the sensors in the group may differ between the settings of two different measurements.

However, it should be realized that some measurements may have same settings. This may be used for providing higher accuracy of each of the individual measurements but may not be necessary for enabling the individual measurements to be extracted.

Each measurement may be performed at a single readout time point. This implies that the sensors may be sampled at the readout time point. However, the sensors may be selected for readout during a time duration allowing a time trace of the measurand to be acquired. Thus, measurement may be performed for at least a short time period to allow the time trace of the measurand to be acquired.

Each sensor may be configured to generate a time-varying modulation of the optical readout signal by receiving a time-varying measurand signal. Thus, the time-varying measurand signal may be converted into a time trace of the measurand in the optical readout signal.

It should be further realized that information of interest may be provided in the time trace of the measurand. Thus, further processing of the measurand may be performed to extract information of interest, such as a frequency within the time trace of the measurand or variation of a frequency over time.

For instance, the sensor device may be used for photo-acoustic imaging. In such case, a pulsed laser is used to emit optical light pulses. The optical light pulses are converted into acoustic pulses upon absorption of the optical light pulses by a sample to be imaged. A total duration for recording the acoustic pulse can be of tens or hundreds of µs. During the measurement of the acoustic pulse, the sensors may be uniquely aligned with respective interrogation wavelengths in order to allow the time trace of a response to the photo-acoustic pulse to be acquired by the sensors.

According to an embodiment, the detector is a photodiode configured to detect intensity of the received optical readout signal.

This implies that a simple component may be used for detecting the optical readout signal. Thanks to the use of series of measurements on linear combinations of individual measurements, the detector need not be able to separately detect the intensity of different wavelengths of the optical readout signal.

According to an embodiment, the waveguide forms part of a plurality of waveguides, wherein each waveguide is associated with a unique group of sensors, wherein a group of waveguides among the plurality of waveguides are configured to simultaneously receive the optical interrogation signal for simultaneous readout of measurements from the groups of sensors associated with the group of waveguides.

This implies that plurality of sensors may be divided into many groups of sensors. Each group may be associated with a respective waveguide. This allows simultaneously detecting measurement signals associated with different groups, which may speed up readout of information for the individual measurements by each of the sensors in the group of sensors.

Each of the waveguides may simultaneously receive the optical interrogation signal. This implies that readout of measurements may be performed for all groups of sensors simultaneously.

However, in some embodiments, a group of waveguides forming a subset of the plurality of waveguides may receive the optical interrogation signal. This may imply that different groups of waveguides need to sequentially receive the optical interrogation signal to allow readout of measurements by all of the sensors.

An optical power in the optical interrogation signal needs to be split onto each of the waveguides in the group of waveguides. Thus, if a large number of waveguides are part of the group of waveguides, the power of the optical interrogation signal may need to be large in order to allow measurements from a plurality of groups of sensors to be read out simultaneously. Hence, providing the optical interrogation signal sequentially to different groups of waveguides may allow a lower power of the optical interrogation signal generated by a light source to be used, avoiding a need of generating a very high power of an optical signal in the light source.

The group of sensors with which a waveguide is associated may correspond to the sensors of a row or a column of an array of sensors. However, it should be realized that the waveguide may be arranged in different manners to be associated with the group of sensors. For instance, the waveguide may be associated with a group of sensors forming part of a row or part of a column, or the waveguide may be associated with a group of sensors forming a sub-array across more than one row and more than one column of the array. Also, the group of sensors need not necessarily be arranged in a regular manner and the sensors in a group need not necessarily be arranged with a particular spatial relation between the sensors.

It should also be realized that a single waveguide may be associated with a group of sensors regardless of the spatial arrangement of the sensors in the group. For instance, the waveguide may be associated with a two-dimensional array of sensors and may thus be configured to follow a path along a row of the sensors, making a turn at an end of the row to continue on a path along a following row in an array, and so on.

The sensor device need not be configured to simultaneously provide the optical interrogation signal to plural waveguides. Rather, the sensor device may alternatively be configured to provide optical interrogation signals sequentially to different waveguides associated with different groups of sensors. This may be used with a single detector for detecting the optical readout signals associated with different waveguides carrying information of readout of measurements. The single detector may sequentially detect the optical readout signal formed based on the optical interrogation signal in respective waveguides.

According to an embodiment, the detector forms part of a plurality of detectors, wherein each detector is associated with a unique waveguide for receiving the optical readout signal.

The optical readout signal received by a detector is formed based on the optical interrogation signal in a waveguide with which the detector is associated. The optical readout signal is thus formed by interaction of the optical interrogation signal with sensors of the group of sensors with which the waveguide is associated.

Thanks to having a plurality of detectors, the optical readout signals representing measurements of a plurality of groups of sensors may be read out simultaneously. This may speed up read out of information from the plurality of sensors.

Each detector may for instance be associated with a waveguide that is associated with a row of sensors. Thus, the sensor device may for instance comprise one detector per row.

According to an embodiment, the sensor device further comprises a splitter configured to receive the optical interrogation signal and configured to split the optical interrogation signal to the group of waveguides.

All of the waveguides may be associated with a single splitter, which may be configured to control which waveguides receive the optical interrogation signal at a particular moment in time.

This implies that the sensor device may be associated with a single light source for generating the optical interrogation signal. The optical interrogation signal may be provided from the light source to the splitter, which then distributes the optical interrogation signal to the waveguides.

According to an embodiment, the sensor device further comprises a light source for generating the optical interrogation signal. Alternatively, the light source may be external to the sensor device and the sensor device may be configured to receive the optical interrogation signal from the external light source.

The light source may for instance be provided by a bank of laser sources configured to generate light at different interrogation wavelengths. The light source may further comprise a combining component configured to receive light from the bank of laser sources and to combine the light into an optical interrogation signal comprising a plurality of separate interrogation wavelengths, which may be output by the combining component into a single input waveguide. The input waveguide may further be connected to the splitter for providing the optical interrogation signal to the splitter.

According to an alternative, the light source may be configured to generate a frequency comb for forming an optical interrogation signal with a plurality of separate interrogation wavelengths.

According to an embodiment, the sensor device further comprises a plurality of control elements, wherein each sensor in the plurality of sensors is associated with a control element of the plurality of control elements for individually controlling the relation between the plurality of separate interrogation wavelengths and the resonance wavelength of optical resonance of the sensor, wherein each control element is individually controllable and configured to control the resonance wavelength of optical resonance of the sensor associated with the control element for tuning the resonance wavelength of the sensor, and wherein the plurality of control elements are configured to provide different settings of the resonance wavelengths of the sensors for the different measurements in the series.

Thus, the sensor device may be configured to use control elements for controlling the resonance wavelengths of the sensors. The control elements may be individually controllable for allowing individual control within a group of sensors of the relation between the interrogation wavelengths and the resonance wavelength.

Each control element may be a unique, individual control element with which the sensor is associated. This implies that each sensor may be accurately and individually controlled.

However, it should be realized that a control element may be associated with a plurality of sensors belonging to different groups. Thus, the control of a plurality of sensors may be provided by one control element. However, within a group of sensors, each sensor is individually controllable to ensure that the different settings of the relations within the group of sensors may be freely provided.

The use of control elements also implies that the sensors within the group of sensors need not be manufactured with static resonance wavelengths that need to differ from each other. This ensures that the sensor device is not limited to a few sensors being arranged in a group in order to achieve unique, differentiable resonance wavelengths during readout of information from the sensors. Rather, the differences in resonance wavelengths within a group of sensors during measurements may be achieved by the control of the control elements.

Further, all of the sensors may even be manufactured with identical optical resonance elements, or with a few different settings of the optical resonance elements. The sensors need not be differentiated from each other during readout by the optical resonance elements being differently manufactured. Rather, the sensors can be differentiated from each other based on the control of the resonance wavelengths provided by the plurality of control elements. Hence, since the sensors may have identical settings of the optical resonance elements or may have only a few different settings of the optical resonance elements, manufacturing of the sensor device is simplified. The manufacturing of the sensor device may thus provide multiple sensors with common characteristics, such that design and layout of the multiple sensors is facilitated. In addition, requirements of tolerances in manufacturing of sensors may be relatively lenient since the sensors may be controlled to be aligned with the interrogation wavelength during readout. Thus, the dynamic control of the resonance wavelength may ensure that the resonance wavelength of the sensor is properly aligned to the interrogation wavelength even if the sensor is not perfectly manufactured to provide characteristics corresponding with nominal characteristics.

The control elements may be any kind of elements that are able to change the resonance wavelength of the optical resonance element of a sensor in a controlled manner. Thus, the resonance wavelength of the optical resonance element of a sensor may be accurately tuned to a desired value using the control element with which the sensor is associated.

Thanks to the sensor device providing controlling of the resonance wavelengths of the sensors, the optical interrogation signal may be provided with fixed interrogation wavelengths. This implies that it may not be necessary to enable tuning of the interrogation wavelengths and, hence, simple laser(s) may be used for generating the optical interrogation signal.

The control element may be configured to affect a characteristic of the optical resonance element so as to cause a change of the optical resonance wavelength. For instance, the control element may be configured to affect a temperature of the optical resonance element which may affect the refractive index of the optical resonance element and may hence in turn affect the resonance wavelength.

However, the control element may be configured to affect the sensor in other manners for controlling the resonance wavelength. The control element may for instance be configured to utilize an electro-optical effect for controlling the resonance wavelength, e.g., by applying an electric field across the optical resonance element, or to utilize a mechano-optical effect for controlling the resonance wavelength, e.g., by physically changing a path length and/or the optical properties of the optical resonance element.

Each control element may be used for tuning the resonance wavelength of the sensor to the desired value. The optical resonance element may have a natural resonance wavelength when not affected by the control element, for instance, by the control element not being active. The sensor may only need to be controlled by the control elements during readout of measurements from the sensor.

According to an embodiment, each control element is configured to tune the resonance wavelength of the sensor associated with the control element for aligning the resonance wavelength with a unique interrogation wavelength of the plurality of separate interrogation wavelengths.

During each measurement in a series of measurements, each sensor within the group of sensors may be associated with a unique interrogation wavelength. Thus, the control element may control the relation of the resonance wavelength and the unique interrogation wavelength. The control element may thus be configured to provide different relations during different measurements in the series such that the measurement signals in the series represent different linear combinations of the individual measurements by the sensors.

According to an embodiment, each control element is configured to tune the resonance wavelength of the sensor associated with the control element for aligning the resonance wavelength with an interrogation wavelength of the plurality of separate interrogation wavelengths by the interrogation wavelength being arranged at a flank of a resonance curve of the resonance wavelength.

This is an efficient manner of sensing the measurand with high sensitivity, since a slight change in the resonance wavelength, as caused by the measurand, may provide a large difference in modulation of optical intensity of the optical interrogation signal.

According to an embodiment, the control element is configured to tune the resonance wavelength of the sensor associated with the control element for providing a positive sign or a negative sign of the measurement of the measurand by the sensor in the linear combination of the measurement signal, wherein the control element is configured tune the resonance wavelength of the sensor associated with the control element for providing the positive sign by the interrogation wavelength being arranged at a flank of negative slope of the resonance curve of the resonance wavelength, and wherein the control element is configured tune the resonance wavelength of the sensor associated with the control element for providing the negative sign by the interrogation wavelength being arranged at a flank of positive slope of the resonance curve of the resonance wavelength.

It is realized that an effect of the measurand on the resonance wavelength may be inverted between the flank of negative slope and the flank of positive slope of the resonance curve. In particular, the flank of negative slope is on a first side of the resonance curve in relation to the peak wavelength and the flank of positive slope is on a second side, opposite to the first side, of the resonance curve in relation to the peak wavelength. This implies that an increase in the resonance wavelength caused by the measurand may cause the interrogation wavelength to be farther from the peak wavelength when the interrogation wavelength is arranged at the flank of negative slope, whereas an increase in the resonance wavelength caused by the measurand may cause the interrogation wavelength to be closer to the peak wavelength when the interrogation wavelength is arranged at the flank of positive slope. Hence, an effect of the measurand on the interaction between the interrogation wavelength with the optical resonance element may be inverted depending on whether the resonance wavelength is tuned for providing the interrogation wavelength at the flank of negative slope or the flank of positive slope, respectively.

The inverted effect of the measurand implies that the control element may provide a factor with a positive sign or a negative sign in the linear combination of the measurement signal. Thanks to enabling both positive signs and negative signs to be used in the linear combination, the sensor device provides a larger degree of freedom in varying the settings for measurements in the series of measurements. This may facilitate providing different linear combinations of the individual measurements for extracting the individual measurements with a high SNR.

According to an embodiment, the sensor device is configured to tune the interrogation wavelengths for controlling the relation between the plurality of separate interrogation wavelengths and the resonance wavelength of optical resonance of the sensors, wherein each interrogation wavelength is associated with a unique sensor and is individually controllable for individually controlling the relation between the interrogation wavelength and the resonance wavelength of the sensor with which the interrogation wavelength is associated, wherein the sensor device is configured to provide different settings of the interrogation wavelengths for different measurements in the series.

Thus, alternatively or additionally to using control elements for controlling the resonance wavelengths, the sensor device may be configured to control the interrogation wavelengths for controlling factors in the linear combinations of the individual measurements by sensors in the series of measurements.

During each measurement in the series of measurements, each sensor within the group of sensors may be associated with a unique interrogation wavelength. Thus, by the sensor device controlling the interrogation wavelength, the sensor device provides control of the relation of the resonance wavelength of a particular sensor and the unique interrogation wavelength.

The sensor device may be configured to tune the interrogation wavelength for aligning the interrogation wavelength with the resonance wavelength of the sensor with which the interrogation wavelength is associated.

The sensor device may further be configured to tune the interrogation wavelength for providing a positive sign or a negative sign of the measurement of the measurand by the sensor in the linear combination of the measurement signal, wherein the sensor device is configured to tune the interrogation wavelength for providing the positive sign by the interrogation wavelength being arranged at a flank of negative slope of the resonance curve of the resonance wavelength, and wherein the sensor device is configured tune the interrogation wavelength for providing the negative sign by the interrogation wavelength being arranged at a flank of positive slope of the resonance curve of the resonance wavelength.

The sensor device may be configured to tune the interrogation wavelength by tuning a light source, such as a laser, generating light at the interrogation wavelength. The sensor device may alternatively be configured to tune the interrogation wavelength by controlling light propagating in a waveguide after being emitted by a light source. The tuning of the interrogation wavelength may be provided before light of different interrogation wavelengths are combined into the optical interrogation signal in order to ensure that the interrogation wavelengths may be individually controlled.

It should be realized that the sensor device may provide opposite signs of the measurement of the measurand by the sensor in the linear combination of the measurement signal by the interrogation wavelength being arranged at different flanks of the resonance curve. However, in some embodiments, a negative sign may be provided by the interrogation wavelength being arranged at the flank of negative slope, and a positive sign may be provided by the interrogation wavelength being arranged at the flank of positive slope.

According to an embodiment, the sensor device is configured to, for each sensor, control the relation between the plurality of separate interrogation wavelengths and the resonance wavelength of the sensor for providing a factor of the measurement of the measurand by the sensor in the linear combination of the measurement signal, wherein the sensor device is configured to control the relation for selecting the factor from a group of values comprising two or more of: +1, 0, and -1.

Thanks to providing factors selected from +1, 0, and -1, the sensor device may enable settings that are vastly different in different linear combinations of the measurement signals in the series of measurements. This facilitates extracting the individual measurements from the measurement signals. Further, the individual measurements may be extracted with a high SNR.

It should be realized that, in other embodiments, the sensor device is configured to control the relation for selecting the factor of individual measurements from non-integer values.

The sensor device is configured to provide an accurate control of the relations between the interrogation wavelengths and the resonance wavelengths of the sensors such that a factor of the measurement of the measurand by each sensor in the linear combination of the measurement signal is known. The known factors may then be used in processing of the series of measurement signals for extracting the individual measurements.

According to an embodiment, the sensor device is configured to perform the series of measurements of the measurand for detecting measurement signals representing a Hadamard matrix of the linear combinations of the individual measurements of the measurand by the sensors in the group of sensors.

This implies that the linear combinations of the individual measurements in the series of measurement signals may be set for providing an optimal SNR.

According to another embodiment, the sensor device is configured to perform the series of measurements of the measurand for detecting measurement signals representing a matrix derived from a Hadamard matrix of the linear combinations of the individual measurements of the measurand by the sensors in the group of sensors.

It should be realized that the Hadamard matrix requires use of factors +1 and -1 in the linear combinations of the individual measurements. However, the factor -1 may not always be possible to provide in controlling of the relation between an interrogation wavelength and a resonance wavelength.

Therefore, as an alternative, a matrix derived from the Hadamard matrix may be used, wherein factors +1 and 0 are used instead in the linear combinations of the individual measurements. For instance, an S-matrix may be used.

The S-matrix may be calculated from a Hadamard matrix by removing a first column and a first row of the Hadamard matrix and converting factors +1 in the Hadamard matrix to 0 and converting factors -1 to +1.

The use of a matrix having only factors +1 and 0 may be associated with simple control, since the factor 0 may be easily applied by turning off a light source that provides the interrogation wavelength. This also implies that a number of interrogation wavelengths needed during each measurement may be smaller. For each measurement, some individual measurements are associated with a factor 0 and, hence, the sensors associated with the factor 0 need not be associated with any interrogation wavelength during the measurement. Thus, the sensor device may be configured to receive an optical interrogation signal with fewer interrogation wavelengths, compared to when a Hadamard matrix is used. The interrogation wavelengths may be associated with different sensors during different measurements in the series of measurements to ensure that the settings to be used for defining the S-matrix may be achieved.

This implies that a light source for generating the optical interrogation signal may be simpler if the S-matrix is used compared to if the Hadamard matrix is used. For instance, if a bank of lasers is used for generating the optical interrogation signal, the number of lasers in the bank of lasers may be reduced.

The S-matrix also provides a high SNR when extracting the individual measurements. However, using the Hadamard matrix a higher SNR may be achieved compared to using the S-matrix.

According to an embodiment, the sensor device further comprises a controller configured to provide control signals for controlling settings of the relations between the plurality of separate interrogation wavelengths and the resonance wavelengths of the sensors for the series of measurements.

The controller may thus be configured to provide control of the settings. The controller may ensure that correct settings are used for the relations of the interrogation wavelengths and the resonance wavelengths of the sensors during the measurements. Further, the controller may thus provide combined information of all of the settings, which may be used for processing of the series of measurement signals.

The controller may be configured to provide the control signals to the control elements, such as heaters, associated with the sensors, for controlling an effect of the control elements on the resonance wavelengths. The control signals may thus control intensity of an output from the control elements. For instance, a control signal may be an electrical signal defining the temperature assumed by the heater for heating the sensor.

The controller may additionally or alternatively be configured to provide the control signals to a light source for controlling tuning of the interrogation wavelengths and/or turning on or off generation of light of one or more interrogation wavelengths.

The controller may be implemented by in hardware or as any combination of software and hardware. For instance, the controller may comprise a central processing unit (CPU), or a graphical processing unit (GPU), comprising software for providing functionality of the controller in a general-purpose processor. Alternatively, the controller may be implemented as firmware arranged in an embedded system of the sensor device. As a further alternative, the controller may be implemented as a special-purpose circuitry for providing specific logical operations. Thus, the controller may be provided in the form of an application-specific integrated circuit (ASIC), an application-specific instruction-set processor (ASIP) or a field-programmable gate array (FPGA).

According to an embodiment, the sensor device further comprises a processor configured to receive the series of measurement signals and configured to process the series of measurement signals for extracting information of the individual measurements of the measurand by the sensors.

Thus, the series of measurement signals acquired by the detector may be provided to a processor for processing the signals. The processor may then be configured to extract information of the individual measurements of the measurand.

The processor may be implemented in hardware or as any combination of software and hardware. For instance, the processor may be implemented in a common CPU or GPU with the controller or in a separate CPU or GPU, wherein the CPU or GPU comprises software for providing functionality of the processor in a general-purpose processor. Alternatively, the processor may be implemented as firmware arranged in an embedded system of the sensor device. As a further alternative, the processor may be implemented as a special-purpose circuitry for providing specific logical operations. Thus, the processor may be provided in the form of an application-specific integrated circuit (ASIC), an application-specific instruction-set processor (ASIP) or a field-programmable gate array (FPGA).

According to yet another alternative, the series of measurement signals detected by the detector may be communicated to a processor that may be external to the sensor device. Thus, the processor may for instance be arranged in a server.

According to a second aspect, there is provided an opto-mechanical sensor device comprising: the sensor device according to the first aspect, wherein each sensor of the plurality of sensors comprises an opto-mechanical element for receiving the measurand, and wherein the opto-mechanical element is configured to be affected by the received measurand so as to change the optical characteristic of the optical resonance element.

Effects and features of this second aspect are largely analogous to those described above in connection with the first aspect. Embodiments mentioned in relation to the first aspect are largely compatible with the second aspect.

The opto-mechanical element may be configured to be mechanically affected by the received measurand so as to change the optical characteristic of the optical resonance element. The measurand may be any property that can mechanically affect the opto-mechanical element. Thus, the measurand may be a force, a pressure, or a mass landing on the opto-mechanical element. The measurand may for instance be provided as an acoustic wave, which provides a varying pressure on the opto-mechanical element.

The opto-mechanical element may for instance comprise a thin membrane which may be deformed based on receiving the measurand.

According to one aspect, there is provided a photo-acoustic imaging device comprising: the sensor device according to the first aspect, wherein each sensor of the plurality of sensors comprises an opto-mechanical element for receiving an acoustic wave forming the measurand, and wherein the opto-mechanical element is configured to be affected by the received acoustic wave so as to change the optical characteristic of the optical resonance element.

The use of the sensor device in the photo-acoustic imaging device enables fast and simultaneous readout of information from a plurality of sensors of the sensor device. In addition, the photo-acoustic imaging device may provide readout of information from the plurality of sensors with a high SNR.

Photo-acoustic imaging may involve using optical pulses to excite acoustic signals in a sample, such as a liquid, gas, solid, tissue. The acoustic signal may then be detected by the plurality of sensors for enabling imaging of the sample.

The photo-acoustic imaging device may use a large number of sensors, enabling imaging of the object with high resolution or imaging of a large size object, while numerous sensors may be read out simultaneously to allow fast acquisition of images of the object.

The photo-acoustic imaging device comprises opto-mechanical elements for receiving an acoustic signal, wherein the acoustic signal affects an optical characteristic of the optical resonance element such that the acoustic signal is detected.

The opto-mechanical elements may be implemented in various manners. For instance, the opto-mechanical elements may comprise a mechanical element that may be formed by membranes, such that each sensor comprises a membrane for receiving an acoustic signal forming the measurand, wherein the membrane is configured to vibrate in dependence of the received acoustic signal, whereby an optical characteristic of the optical resonance element arranged on or in the vicinity of the membrane may be affected. According to an alternative, the opto-mechanical element may comprise a disk configured to bend upon receiving an acoustic signal or a Fabry-Pérot sensor.

According to a third aspect, there is provided a method for acquiring information of a measurand, said method comprising: performing a series of measurements of the measurand by a plurality of sensors, wherein each sensor comprises an optical resonance element, wherein an optical characteristic of the optical resonance element is configured to be affected by the measurand to be measured by the sensor; and for each measurement in the series of measurements: receiving an optical interrogation signal comprising a plurality of interrogation wavelengths in a waveguide associated with a group of sensors among the plurality of sensors; for each sensor in the group of sensors, individually controlling a relation between the plurality of interrogation wavelengths and the resonance wavelength of the sensor; and detecting a measurement signal based on an intensity of an optical readout signal, which is based on interaction of the optical interrogation signal with each of the sensors in the group of sensors, wherein the measurement signal represents a linear combination of individual measurements of the measurand by the sensors in the group of sensors, wherein the controlling of the relation between the plurality of interrogation wavelengths and the resonance wavelength of each sensor provides different settings of the relations for different measurements in the series, wherein the measurement signals in the series of measurements signals represent different linear combinations of the individual measurements of the measurand by the sensors in the group of sensors.

Effects and features of this third aspect are largely analogous to those described above in connection with the first and second aspects. Embodiments mentioned in relation to the first and second aspects are largely compatible with the third aspect.

According to the method, the use of the linear combination of the individual measurements in the series of measurement signals may allow the individual measurements to be extracted with a high SNR. In particular, the linear combinations may be selected such that an SNR of the individual measurements may be higher than a corresponding SNR based on reading out each sensor individually, one at a time.

### Brief description of the drawings

The above, as well as additional objects, features, and advantages of the present inventive concept, will be better understood through the following illustrative and non-limiting detailed description, with reference to the appended drawings. In the drawings like reference numerals will be used for like elements unless stated otherwise.
Fig. 1a is a schematic perspective view of a sensor according to an embodiment with a partially cut-away membrane to illustrate all features of the sensor.
Fig. 1b is a schematic top view of the sensor in Fig. 1a with a cut-away membrane.
Fig. 1c is a schematic graph illustrating detection of intensity changes of a transmitted signal based on a measurand acting on the sensor.
Fig. 2 is a schematic graph illustrating a transmission curve of a sensor and a self-thermo-optic effect of altering the transmission curve of the sensor.
Fig. 3 is a schematic graph illustrating providing measurements based on detecting the measurand by the sensor to an optical interrogation signal, wherein the measurements are provided with different signs depending on relation of the optical interrogation signal to the transmission curve of the sensor.
Fig. 4 are schematic graphs of noise spectrum of the sensor in air and water, respectively.
Fig. 5a is a schematic view of a sensor device illustrating a multiplexing scheme allowing reading out measurements, where the multiplexing scheme is provided as an example for comparing to multiplexing schemes according to aspects of the present description.
Fig. 5b is a schematic graph illustrating a limited number of sensors that may be read out using the multiplexing scheme of the sensor device of Fig. 5a.
Fig. 6 is a schematic view of a sensor device according to an embodiment.
Fig. 7 is a schematic view of linear combinations of individual measurements in a series of measurements according to an embodiment.
Fig. 8 is a flow chart of a method according to an embodiment.

### Detailed description

According to the first aspect, there is provided a sensor device adapted for readout of measurements from a plurality of sensors. The sensor device will be described below in relation mainly to measurements of a measurand being formed by an acoustic signal. However, it should be realized that the sensor device may be used for readout of measurements of other types of measurands.

The use of optical Micro and Nano-Electro-Mechanical Systems (MOEMS and NOEMS) sensor devices, where the measurand measurement is carried out through an optical readout, is becoming widespread. Such sensor devices have multiple sensing applications such as chemical, mass, biological, pressure, temperature, force, and stiffness sensing.

The sensor devices comprise an optical resonance element, such as ring, disk, sphere, Fabry-Pérot, or photonic crystals geometries. The measurand of interest modifies the sensor optical reflection or transmission response by changing a mechanical state of the sensor, which may be referred to as an opto-mechanical interaction.

Opto-mechanical ultrasound sensor (OMUS) devices is an example of above discussed sensor devices and are used for ultrasound detection. Herein, readout schemes will be discussed for OMUS devices. However, such schemes can be used for other MOEMS/NOEMS sensor device architectures and applications. In addition, the manner of multiplexed readout of information could be generalized to other types of optical sensors, not necessarily based on the opto-mechanical interaction.

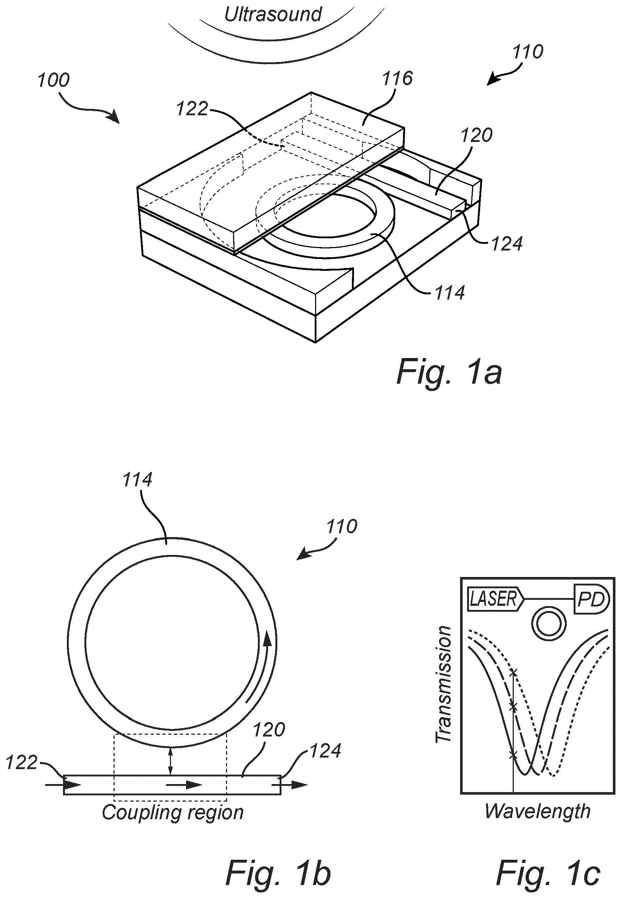

Referring now to Figs 1a-b, an OMUS pressure sensor 110 according to an embodiment may be based on a membrane 116 with a circular waveguide 114, or ring resonator forming an optical resonance element of the sensor 110, positioned below the membrane 116. The ring resonator 114 may be optically coupled to the membrane 116 such that an optical characteristic, e.g., a resonance wavelength, of the ring resonator 114 is changed in dependence of movement of the membrane 116. The ring resonator 114 may further be optically coupled to a waveguide 120, which may follow a straight path as shown in Figs 1a-b. The straight waveguide 120 allows light forming an optical interrogation signal to be sent into the ring resonator 114 via an input port 122 and allows transmitted intensity to be read out via a through port 124.

When the light coupled to the ring resonator 114 via the straight waveguide 120 is such that the optical path of the ring resonator 114 is a specific integer multiple of the wavelength, the ring resonator 114 is on resonance. The number of wavelengths m fitting in the optical path of the ring may be called the order of the mode. For light at the resonance wavelength of a confined mode in the ring resonator 114, destructive interferences occur between the light which has coupled to the ring resonator 114 and the light that passes directly through the straight waveguide 120. This implies that the total light intensity that passes through the straight waveguide 120 when the ring resonator 114 is at resonance decreases with respect to a case when the ring resonator 114 is off resonance. This results in a dip in a measured transmission curve at resonance.

As shown in Fig. 1c, when detecting pressure waves, such as acoustic waves, motion of the membrane 116 leads to changes in a gap between the ring resonator 114 and the membrane 116, which changes an effective refractive index of the mode confined in the ring resonator 114, which in turn changes the resonant wavelength of the ring resonator 114. The ultrasound pulse incident on the membrane 116 can be transduced into the optical interrogation signal propagating in the straight waveguide 120 by setting a readout laser providing the optical interrogation signal to a flank of optical resonance of the ring resonator 114. As illustrated in Fig. 1c, the readout laser may be set on the point of the flank where transmission slope is maximal, to maximize the opto-mechanical transduction sensitivity. Variations in optical intensities may be measured with a photodiode, enabling to acquire an electrical signal representing the measurand.

During photo-acoustic imaging, an object that is to be imaged is the source of the acoustic waves being measured. The object is excited by a pulsed laser, which is different from the readout laser. The OMUS sensor device 100 comprises a plurality of sensors 110 that are used for recording multiple time traces from different sensors 110. These time traces may then be used to obtain a 3D image of the object.

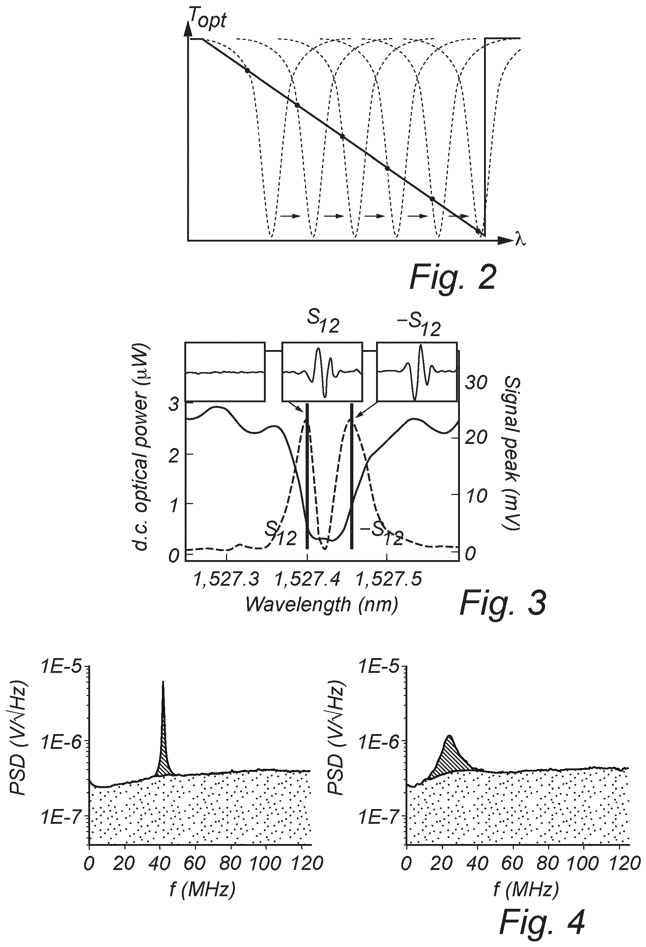

Referring now to Fig. 2, the transmission curve of the OMUS sensor 110 has a Lorentzian shape that can be altered by a self-thermo-optic effect. The self-thermo-optic effect may occur when high input laser powers are coupled to the OMUS sensor 110 to read out the measurement by the OMUS sensor 110. Indeed, when some of the light of the optical interrogation signal is absorbed, it causes an increase of temperature inside the ring resonator 114. As the refractive index of the ring resonator 114, which may for instance be formed by a semiconductor material, may depend on the temperature of the material (which is a thermo-optic effect), the effective refractive index of the confined optical mode in the ring resonator 114 depends on the coupled optical power. The effect is referred to herein as a self-thermo-optic effect to indicate that it is the optical interrogation signal, that is, the readout laser, which generates this effect.

When sweeping the transmission curve of a ring resonator 114 around one of its resonance wavelengths, the measured resonance curve is not Lorentzian, as further represented in Fig. 2. Indeed, when tuning the readout laser towards longer wavelengths (i.e., towards the right-hand side of Fig. 2), the coupled power into the confined mode increases (solid line, Fig. 2). This causes the effective refractive index to increase, and hence the resonance wavelength is increased, or red shifted, (successive Lorentzian curves, dashed lines, Fig. 2). Hence, the optical mode moves away from a wavelength of the readout laser, until the readout laser is at the bottom of the optical mode (Lorentzian curve farthest to the right, Fig. 2). In this case, an increase in the wavelength of the readout laser decreases the coupled optical power, which cools down the mode, which further decreases the resonance wavelength, and so on. Hence the optical mode abruptly moves back to its initial spectral position before being heated by the laser (Lorentzian curve farthest to the left, Fig. 2). This sequence of events results in a triangular shaped transmission curve.

Hence, it is clear that because of the triangular shape of the optical mode due to the self-thermo-optic effect, only the left flank of the transmission curve (i.e., wavelengths on the transmission curve shorter than a peak value of the transmission curve) may be accessible to transduce optically the mechanical motion. Further, the ring resonator 114 is advantageously interrogated with the laser sweeping the transmission curve from the left hand-side (i.e., from shorter wavelengths towards longer wavelengths) of the resonance wavelength.

Referring now to Fig. 3, sensing of a measurand when the self-thermo-optic effect is not dominant will be described.

In this case both the left and the right flank of the Lorentzian resonance curve can be used to readout the signal generated by the OMUS sensor 110. It should be noted that the slopes of the transmission curve are of opposite sign when compare the left flank of the resonance curve with respect to the right flank of the resonance curve. The consequence of this difference in the signs of the slopes is that the sign of the measured signal can be inverted depending on the flank of the optical resonance at which the interrogation wavelength is aligned.

Fig. 3 illustrates the resonance curve in solid line and further illustrates a sensitivity of variations in alignment of an interrogation wavelength to the resonance curve in dashed line. Fig. 3 further illustrates in solid, vertical lines arrangement of interrogation wavelengths at a first peak of sensitivity at the left flank of the optical resonance and at a second peak of sensitivity at the right flank of the optical resonance.

As illustrated in Fig. 3, when light at the interrogation wavelength is turned off or when the interrogation wavelength is not aligned with the resonance wavelength, a measurement of the measurand by the sensor will not affect the optical interrogation signal (left-most inserted graph at top of Fig. 3). When the interrogation wavelength is aligned on the left flank of the optical mode, a signal s₁₂ (center inserted graph at top of Fig. 3) is transferred to the optical interrogation signal representing a received measurand by the sensor. When the interrogation wavelength is instead aligned on the right flank, the measured signal will take an inverted appearance, that is, and a signal -s₁₂ (right-most inserted graph at top of Fig. 3) is transferred to the optical interrogation signal representing a received measurand by the sensor.

It should be realized that the sensor device 100 typically comprises a large plurality of sensors 110. Thus, capturing signals representing measurements coming from different sensors 110 is desired for the implementation of the sensor device 100 for real-life applications.

For example, in photo-acoustic imaging, multiple time traces may be recorded with ultrasound sensors 110 from multiple locations to reconstruct an image.

Another example is mass sensing based on opto-mechanical mass detectors. To this end, multiple mass landing events need to be recorded from highly sensitive and high frequency opto-mechanical mass sensors, to maximize a capture cross-section of the sensor device. This is particularly useful in biosensing and spectroscopy applications. Increasing the number of sensors decreases measurement time and a required quantity of analyte. In addition, it enables to get spatial mapping of the mass landing events.

To read, or multiplex/demultiplex, information of measurements coming from multiple MOEMS/NOEMS sensors 110, different degrees of freedom can be used. For instance, wavelength multiplexing may be used, wherein each sensor 110 is labelled with a unique readout interrogation wavelength. Hence, by coupling to each sensor a unique interrogation wavelength, and by separating the interrogation wavelengths with a wavelength demultiplexer, the signals representing measurements originating from different sensors 110 may be retrieved. Additionally, or alternatively, time multiplexing may be used, wherein each sensor 110 is read sequentially in time. The sensor 110 may thus be identified by the time interval at which measurements are read out.

To increase the number of sensors 110 that can be interrogated, a multiplexing/demultiplexing scheme may be used in a controlled manner, for example by using the degrees of freedom described above. The read-out scheme should enable parallel (i.e., simultaneous) readout of sensors 110, while preserving a simple measurement scheme.

Hereinafter, a sensor device 100 exploiting the degrees of freedom providing efficient readout schemes that may be used with large arrays of sensors 110 is described. The readout schemes are provided, while preserving reasonable numbers of readout lasers, photodiodes for detecting an optical interrogation signal having interacted with sensors 110, and fibres or waveguides for guiding optical signals. In addition, the sensor device 100 may provide a simple photonic integrated circuit (PIC) architecture providing guiding of the optical interrogation signal.

Referring now to Fig. 4, noise sources of the sensors 110 will be discussed. Opto-mechanical sensors 110 are sensitive to multiple noise sources, such as laser noise, i.e., noise in the optical interrogation signal, photodiode noise, and thermomechanical noise. Often, the dominant noise sources are the photodiode noise and the thermomechanical noise.

Photodiode noise corresponds to the noise generated by a photodetector used to readout the signal coming from the opto-mechanical sensor 110.

Thermomechanical noise (TMN) corresponds to the noise generated by the opto-mechanical sensor 110 due to the Brownian motion of the mechanical degree of freedom (i.e., the membrane 114 in the case of an OMUS sensor 110). Such incoherent motion is transduced by the opto-mechanical interaction and is part of the opto-mechanical sensor noise.

Fig. 4 illustrates the noise power spectral densities (PSD) of an OMUS sensor 110 arranged in air, left graph, and water, right graph. TMN is clearly identified as a Lorentzian peak centered at the resonance frequency of the membrane (regions with dashed pattern). Far from the resonance frequency, the noise is dominated by the noise from the photodiode (regions with dotted pattern).

OMUS sensors 110 present high capabilities in measurement of weak mechanical vibrations. This feature is quantified by a noise equivalent pressure (NEP) of the OMUS sensors 110, which corresponds to a pressure wave amplitude over a band of 1 Hz giving a SNR equal to 1. The NEP can reach values down to few $mPa / \sqrt{Hz}$ for OMUS sensors.

The NEP for OMUS sensors may be limited mainly by the photodiode noise and TMN. Decreasing the NEP value, that is, increasing the SNR obtained during the measurement of a mechanical wave would therefore allow to improve the performances of the sensors during, e.g., photoacoustic imaging experiments.

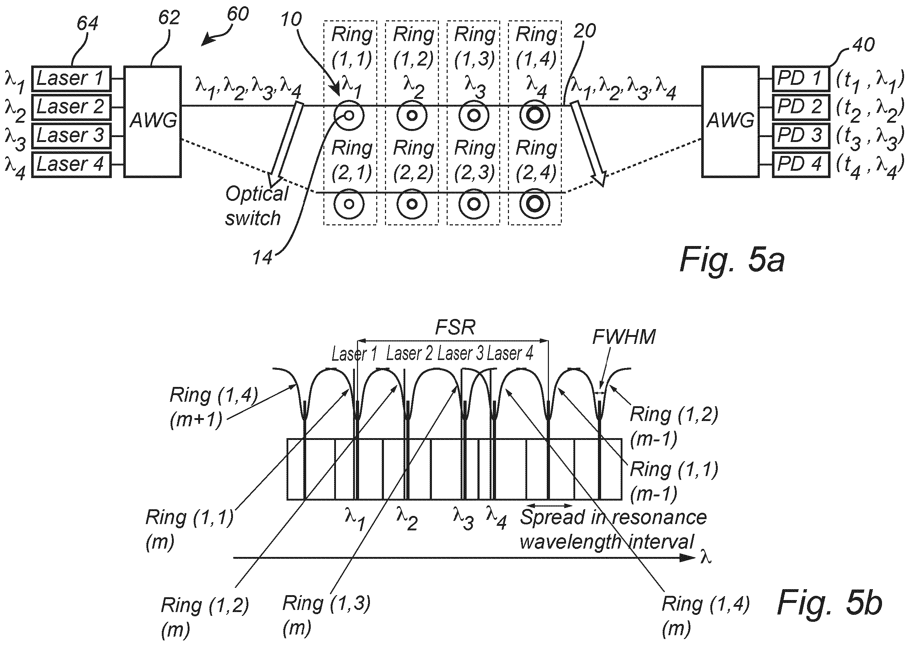

Referring now to Fig. 5a, a readout scheme for readout of a plurality of sensors 10 will first be described for providing a comparison to readout schemes according to aspects of the present description.

For instance, as mentioned above, in photo-acoustic imaging applications, readout of a large array of opto-mechanical sensors 10 may be needed in an efficient manner. The array comprises a plurality of OMUS sensors 10 that may be designed to respond to specific acoustic frequencies. The acoustic time traces recorded by the array of differently positioned sensors 10 allow reconstruction of photo-acoustic images.

Each OMUS sensor 10 can be identified by its row and column number in the array. If the array comprises P rows and P columns, then the total number of OMUS sensors 10 is equal to N = P × P. The number of sensors 10 to be read out can scale from tens up to thousands of sensors 10, depending on the application. The OMUS sensors 10 present in one row of the array may be evanescently coupled to the same waveguide 20, which may thus be associated with a group of sensors arranged in one row of the array. This provides a simple layout of the waveguide 20, which carries the optical interrogation signal to the sensors 10, in relation to the sensors 10.

As shown in Fig. 5a, each ring resonator 14 may column-wise be fabricated with a slightly different diameter (size differences are exaggerated in Fig. 5a for illustration purposes), allowing to have a slightly different optical mode resonance wavelength for each sensor 10 on a given row.

To read out measurements from such an array of sensors 10, readout schemes are needed that present realistic numbers of required readout light sources, photodetectors, and simple PICs. One strategy as illustrated in Fig. 5a is to implement readout multiplexing schemes enabling to read out a row of the array using limited numbers of light sources and photo detectors.

An arrayed waveguide grating (AWG) component 62 and a bank of laser sources 64 providing different interrogation wavelengths may be used for creating a combined light source 60 providing an optical interrogation signal with a plurality of separate interrogation wavelengths. Each laser line is aligned with a different optical resonance wavelength of the rings on the row. After passing the sensors 10 evanescently coupled to the waveguide 20, the interrogation wavelengths can be split again and be individually measured by a detector 40.

Combining this wavelength multiplexing strategy with time multiplexing, an entire array of sensors 10 can be readout. An optical switch may be used for coupling the optical interrogation signal sequentially in time into waveguides 20 associated with different rows of the array, such that the optical interrogation signal is switched between consecutive rows once the measurement on a previous row is completed.

This wavelength multiplexing/demultiplexing scheme allows identifying each sensor 10 based on its associated resonance wavelength (column number) and time step (row number) at which it was measured.

As shown in Fig. 5b, there is a wavelength interval available in which the interrogation wavelengths are to be provided, due to use of circular waveguides 14 providing an optical resonance element of the sensors 10. For example, if the diameter of the ring resonators 14 are increased over one row to increase the resonance wavelength of a confined mode of order m for each ring, the confined mode of order m for a ring will eventually reach a resonance wavelength of the confined mode of order m - 1 for a first ring in the row. A wavelength difference between the mode of order m and the mode of order m - 1 is called the free spectral range (FSR) of a ring. Thus, for each ring with a given resonance wavelength for the mode with a given order m, modes corresponding to higher and lower order values are also introduced (i.e., harmonics). Hence there is a limited wavelength interval in which the resonance wavelengths of the ring resonators should be placed. This wavelength interval is given by the FSR.

In addition, an optical mode of a ring resonator needs to be spectrally separated in the wavelength domain from optical modes of other ring resonators. The spectral space occupied by a resonance wavelength can be quantified for example by a full width half maximum (FWHM) of the mode. Hence, a limited number of different diameters of rings can be placed on an FSR.

If t_{read} is the readout time for a sensor 10 in Fig. 5a, then the total matrix readout time is P × t_{read} (that is, each sensor 10 in the matrix is readout once). In other words, P time steps (i.e., the number of rows in the matrix), are necessary to read out the full matrix.

The laser optical power needed to readout the signal generated by one OMUS sensor 10 is referred to as P₀. A power of P₀ for each of the P interrogation lasers is thus necessary to readout the matrix row by row.

The SNR of the signal measured by each OMUS sensor 10 for such readout scheme may be analyzed for comparison to the readout schemes discussed below. The considered noise sources are the photodiode noise and TMN, characterized by their variances ${σ}_{PD}^{2}$ and ${σ}_{TMN}^{2}$, respectively. These noise variances may be assumed to be constant over the different acquired signals and over the different measured detectors 40, for simplicity. The noises from the different sensors 10 considered here are assumed to be uncorrelated, as well as noises with the signals. The noise is considered here as a random variable with zero mean. Hence, the total noise of the system is equal to the sum of all the noise variances.

In this case, as each opto-mechanical sensor 10 is readout once by one photodiode, the SNR of the measured signal for a readout sensor in row i and column j, i.e., at location (i, j) is given by: ${SNR}_{i , j} = \frac{{s}_{i , j}}{\sqrt{{σ}_{TMN}^{2} + {σ}_{PD}^{2}}} ,$ where s_{i,j} is the signal amplitude measured by the sensor at location (i, j) in the matrix.

Another approach is to carry out a column-by-column readout of the full matrix. In such example, a single readout laser is used. The readout laser power is divided by a power splitter in all the rows of the matrix and its wavelength is tuned to the rings belonging to one column. Each row is coupled to a different photodiode, allowing to identify the readout row number. By changing the laser wavelength, a sequential column by column readout is carried out. Hence, in this case each opto-mechanical sensor is identified by the readout time and the photodiode number which read out the generated signal.

In this example, for a P × P sensors square matrix, 1 laser emitting a power P × P₀ and P photodiodes are necessary for a total matrix readout time of P × t_{read}. Notice that in contrast to a row-by-row readout, no active optical elements such as an optical switch are necessary.

Again, as each opto-mechanical sensor is readout once by one photodiode, in this case the same SNR is provided as for a row-by-row readout described before, that is: ${SNR}_{i , j} = \frac{{s}_{i , j}}{\sqrt{{σ}_{TMN}^{2} + {σ}_{PD}^{2}}} ,$ where s_{i,j} is the signal amplitude measured by the sensor at location (i, j) in the matrix.

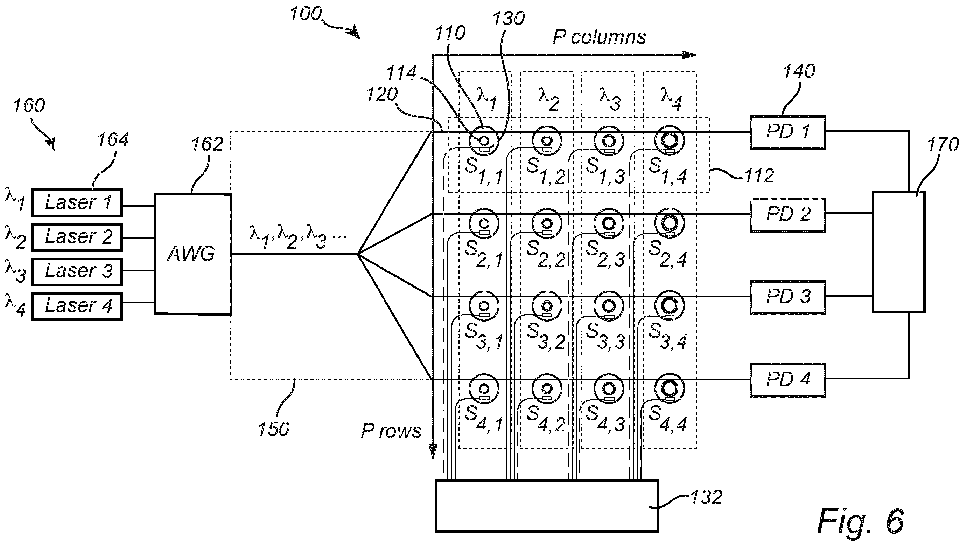

Referring now to Fig. 6, a sensor device 100 according to an embodiment will be described for providing improved SNR of readout of measurement signals compared to the readout schemes discussed above.

Fig. 6 illustrates that the sensor device 100 comprises a light source 160 having a bank of lasers 164 for providing separate interrogation wavelengths. Signals from each of the lasers 164 may be received by an optical component, such as an AWG 162, which may be configured to receive signal components from each of the lasers 164 and further be configured to combine the signal components into an optical interrogation signal comprising a plurality of interrogation wavelengths. The optical interrogation signal may further be provided to an optical splitter 150, which may be configured to split optical power of the optical interrogation signal into a plurality of waveguides 120 for simultaneously providing the optical interrogation signal to the plurality of waveguides 120.

It should be realized that the combining of the signal components and splitting of the optical interrogation signal into plural waveguides 120 may be achieved in other ways.

Each waveguide 120 is associated with a group of sensors 112, in this case a group of sensors 112 arranged along a row of the plurality of sensors. Each waveguide 120 is configured to simultaneously receive the optical interrogation signal for simultaneous readout of measurements from the groups of sensors 112 associated with the waveguides 120.

The waveguide 120 passes the sensors 112 and the optical interrogation signal interacts with the optical resonance elements 114 of the sensors 110 for affecting the optical interrogation signal. Thus, an optical readout signal may be formed at an end of the waveguide 120 having passed all of the sensors 110 in the group of sensors 112. Each signal component of the optical readout signal provides information of a measurand measured by the sensor 110 having a resonance wavelength aligned with the interrogation wavelength of the signal component.

The optical readout signal is provided to a detector 140. The detector 140 may be photodiode configured to detect intensity of the received optical readout signal.

The sensor device 110 comprises a plurality of detectors 140. Each detector 140 is associated with a unique waveguide 120 for receiving the optical readout signal from the waveguide 120 with which it is associated.

When interrogating all the sensors 110 in a row, each interrogation wavelength couples to a single opto-mechanical sensor 110 and all the signals are then measured by the same photodiode 140. Since each ring 114 provides a different resonance wavelength and therefore is associated with a different interrogation wavelength, interferences between the different individual measurements coming from different sensors 110 can be neglected as discussed before. Hence, the photodiode 140 measures the coherent sum of all the optical intensities transmitted when the optical interrogation signal passes each ring.

Therefore, the total measurement signal measured by the detector 140 is the sum of each signal generated by each sensor. For example, for a first photodiode PD 1, the measurement signal s_{measPD₁t ₁} at timestep t₁, where all the interrogation wavelengths are aligned to the left flanks of the optical resonances of the interrogated sensors, is: ${s}_{meas PD 1 , {t}_{1}} = {s}_{1 , 1 , {t}_{1}} + {s}_{1 , 2 , {t}_{1}} + {s}_{1 , 3 , {t}_{1}} … = {\sum }_{k = 1}^{P} {s}_{1 , k , {t}_{1}} ,$ where s_{1,1,t₁} is the individual measurement of sensor at row 1 and column 1 at timestep t₁, *s*_{1,2,t₁} is the individual measurement of sensor at row 1 and column 2 at timestep t₁, and so on. The measurement signal is thus a linear combination of the individual measurements of the measurand by the sensors 110 in the group of sensors 112 with which the waveguide 120 is associated.

A minus sign on each term can be added if the measured flank is changed from the left one to the right one (cf. Fig. 3). For example, if this is done for the sensor in row 1 and column 2, i.e., for the signal s_{1,2,t₁}, the linear combination of the measurement signal becomes: ${s}_{meas PD 1 , {t}_{1}} = {s}_{1 , 1 , {t}_{1}} - {s}_{1 , 2 , {t}_{1}} + {s}_{1 , 3 , {t}_{1}} + ⋯$

The sensor device 100 is configured to acquire different linear combinations of the individual measurements at different timesteps, where the ± signs are swapped by choosing different flanks of the optical resonances for the interrogated sensors. Thus, the sensor device 100 is configured to perform a series of measurements of the measurand by the groups of sensors, wherein different settings are used for detecting a series of measurement signals, wherein the measurement signals in the series represent different linear combinations of the individual measurements of the measurand by the sensors.

In addition, a 0 sign can also be applied by not measuring the opto-mechanical sensor 110. This can be done by switching off the readout laser or by moving out of resonance the interrogation wavelength with respect to the optical resonance of the opto-mechanical sensor 110.

Each sensor 110 may be associated with a control element 130 which is configured to control the resonance wavelength of optical resonance of the optical resonance element, such as the ring resonator 114, of the sensor 110. The control element 130 may be configured to change an optical characteristic of the ring resonator 114, such as changing a refractive index of the ring resonator 114.

The control element 130 may for instance be provided as a heater, which may be controlled by a small current. The heater may thus be configured to heat the ring resonator 114 such that the refractive index of the waveguide of the ring resonator 114 is changed due to the thermo-optic effect. This causes the resonance wavelength of the ring resonator to be shifted to a longer wavelength.

Thus, the alignment of the resonance wavelength of the ring resonator 114 with the interrogation wavelength provided by the read-out laser may be provided by controlling the resonance wavelength of the ring resonator 114 using the control element 130.

The control elements 130 may thus be used for providing different relations between the interrogation wavelengths and the resonance wavelengths of the sensors 110 for different measurements in the series. During each measurement, each control element 130 may be configured to tune the resonance wavelength of the sensor 110 associated with the control element 130 for aligning the resonance wavelength with a unique interrogation wavelength of the plurality of separate interrogation wavelengths.

Each control element 130 may be configured to tune the resonance wavelength of the sensor 110 associated with the control element 130 for aligning the resonance wavelength with an interrogation wavelength of the plurality of separate interrogation wavelengths by the interrogation wavelength being arranged at a flank of a resonance curve of the resonance wavelength. This may provide high sensitivity of reading out the measurement of the measurand.

As discussed above in relation to Fig. 3, each control element may be configured to tune resonance wavelength of the sensor 110 for arranging the interrogation wavelength at a left flank of the resonance curve for providing a positive sign to the individual measurement signal in the linear combination or for arranging the interrogation wavelength at a right flank of the resonance curve for providing a negative sign to the individual measurement signal in the linear combination.

It should be further realized that in addition to, or alternatively to using the control elements 130 for controlling the resonance wavelengths of the optical resonance elements 114 of the sensors 110, the sensor device 100 may be configured to tune the interrogation wavelengths for controlling the relation between the interrogation wavelengths and the resonance wavelengths.

The sensor device 100 may comprise a controller 132 configured to provide control signals for controlling settings of the relations between the interrogation wavelengths and the resonance wavelengths of the sensors 110 for the measurements. The controller 132 may send the control signals to the control elements 130 and/or to the lasers 164 for controlling the interrogation wavelengths.

The controller 132 may thus provide factors for the individual measurements of the measurand by the sensors 110 in the linear combination of the measurement signal. For instance, the controller 132 may be configured to provide control signals for selecting the factor from values of +1, 0, and -1.

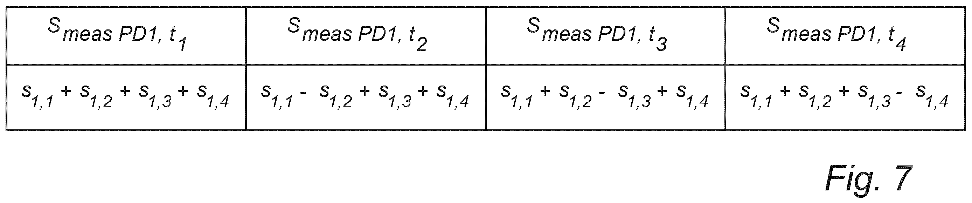

Referring now to Fig. 7, linear combinations of measurement signals acquired by the first photodiode PD1 associated with the first row of sensors at four different time steps t₁, t₂, t₃, t₄, are illustrated. The measurement signal acquired at the time steps are indicated as s_{measPD₁t₁}, s_{measPD₁t₂}, s_{measPD₁t₃}, and s_{measPD₁t₄}, respectively. The measurement signals are provided as different linear combinations of the contributions from four different sensors, wherein the individual measurement by each sensor is represented as s_{1,1} for the sensor in row 1 and column 1, as s_{1,2} for the sensor in row 1 and column 2, as s_{1,3} for the sensor in row 1 and column 3, and as s_{1,4} for the sensor in row 1 and column 4. As shown in Fig. 7, a factor +1 or -1 is provided for the individual measurements and the factors for the respective individual measurements are different at different time steps.

It should be realized that corresponding measurement signals may be simultaneously acquired by each photodiode of the sensor device 100 receiving optical readout signals from different waveguides 120. However, for simplicity, only the measurement signals acquired by one photodiode 140 is considered here.

The sign decomposition of the factors provided in the linear combinations of the individual measurements in a series of measurement signals may be represented in the form of the sign matrix, or multiplexing matrix **A,** where the rows correspond to different time intervals, whereas the columns correspond to a different sensor on the considered row. In the case illustrated in Fig. 7, for PD 1, such matrix is: $A = \left[\begin{matrix}1 & 1 & 1 & 1 \\ 1 & - 1 & 1 & 1 \\ 1 & 1 & - 1 & 1 \\ 1 & 1 & 1 & - 1\end{matrix}\right] .$

By acquiring different s _{PD1} signals obtained for the same photoacoustic pulse, but with different signs applied to the individual measurements, this matrix decomposition can be used to isolate time traces of the signals of interest s_{1,1}(t), s_{1,2}(t), s_{1,3}(t) ... This can be done by carrying out linear combinations of the measured signals s_{meas PD1,tᵢ} measured at different timesteps tᵢ. For example, s_{1,1}(t) can be recovered by carrying out the linear combination: ${s}_{1 , 1} \left(t\right) = \frac{- {s}_{meas PD 1 , {t}_{1}} + {s}_{meas PD 1 , {t}_{2}} + {s}_{meas PD 1 , {t}_{3}} + {s}_{meas PD 1 , {t}_{4}}}{2} .$

This can be explained by noting the different signals generated by the 4 different sensors in the first row as the signal vector: $s = {\left[\begin{matrix}{s}_{11} \\ {s}_{12} \\ {s}_{13} \\ {s}_{14}\end{matrix}\right]}_{4 \times 1} .$

Hence, the signal measured by the photodiode PD 1 at the different measurement time steps is given by: ${s}_{meas PD} = As = {\left[\begin{matrix}{s}_{1 , 1} + {s}_{1 , 2} + {s}_{1 , 3} + {s}_{1 , 4} \\ {s}_{1 , 1} - {s}_{1 , 2} + {s}_{1 , 3} + {s}_{1 , 4} \\ {s}_{1 , 1} + {s}_{1 , 2} - {s}_{1 , 3} + {s}_{1 , 4} \\ {s}_{1 , 1} + {s}_{1 , 2} + {s}_{1 , 3} - {s}_{1 , 4}\end{matrix}\right]}_{4 \times 1} = {\left[\begin{matrix}{s}_{meas PD 1 , {t}_{1}} \\ {s}_{meas PD 1 , {t}_{2}} \\ {s}_{meas PD 1 , {t}_{3}} \\ {s}_{meas PD 1 , {t}_{4}}\end{matrix}\right]}_{4 \times 1} ,$ where each row in **s_{meas PD}** corresponds to a signal measured by PD1 at different measurement time steps.

The inverse **A⁻¹** of the multiplexing matrix may also be calculated. In this example, the inverse matrix is equal to: ${A}^{- 1} = \frac{1}{2} \left[\begin{matrix}- 1 & 1 & 1 & 1 \\ 1 & - 1 & 0 & 0 \\ 1 & 0 & - 1 & 0 \\ 1 & 0 & 0 & - 1\end{matrix}\right] .$

Further, this allows recovering the individual measurements using a relation: ${s}_{rec} = {A}^{- 1} {s}_{meas PD} = {A}^{- 1} As = s .$

Multiplying **s_{meas PD}** by **A⁻¹** thus allows recovering the individual measurement signals contained in **s** and store them in a **s_{rec},** vector. It is the linear combination contained in the first row of **A⁻¹** that was used to recover s_{1,1}(t) as presented above.

From this example, it is clear that such method can be generalized for an opto-mechanical sensor 2D matrix of any size P × P, by defining a P × 1 signal vector: ${s}_{q} = {\left[\begin{matrix}{s}_{q , 1} \\ {s}_{q , 2} \\ ⋮ \\ {s}_{q , P}\end{matrix}\right]}_{P \times 1} ,$ where q is the row number in the 2D matrix, and the row number in this vector is the column number in the matrix. ${s}_{meas {PD}_{q}} = As = {\left[\begin{matrix}{s}_{meas {PD}_{q} , {t}_{1}} \\ {s}_{meas {PD}_{q} , {t}_{2}} \\ ⋮ \\ {s}_{meas {PD}_{q} , {t}_{P}}\end{matrix}\right]}_{P \times 1}$

An invertible multiplexing matrix **A** of size P × P can be defined, comprising terms with factors +1, -1 and 0. The terms included in the multiplexing matrix are applied by controlling the interrogation wavelengths of the readout lasers with respect to the resonance wavelengths of the sensors, as explained before. The inverse **A⁻¹** of the matrix can be calculated and the sensors signals can be recovered by considering that: $s = {A}^{- 1} {s}_{meas PD} .$

The series of measurement signals may be provided to a processor 170, which may be connected to the detectors 140 for receiving the measurement signals. The processor 170 may be configured to process the series of measurements as discussed above for extracting information of the individual measurements of the measurand by the sensors 110.

The superposition of signals allows implementing of Hadamard multiplexing schemes. Thus, the sensor device 100 may be configured to control the relations between the interrogation wavelengths and the resonance wavelengths of the sensors 110 for detecting measurement signals representing a Hadamard matrix of the linear combinations of the individual measurements.

Hadamard multiplexing schemes allow giving an optimal SNR improvement through linear combinations of measured signals (by reducing by a factor P the photodiode noise variance contribution to the total measurement noise, where P is the number of sequential measurements done by each photodiode), with respect to the case where only one sensor is readout by each photodiode, as discussed above in relation to Fig. 5a. This is possible when +1 and -1 terms can be used for making the linear combinations.

For providing a detailed example, sensors arranged in a square matrix is considered, wherein the matrix comprises P = 2ⁿ rows and columns, where n is an integer. The following basic Hadamard matrix is used: ${H}_{2 \times 2} = \left[\begin{matrix}1 & 1 \\ 1 & - 1\end{matrix}\right] .$

Based on the basic Hadamard matrix, a 2ⁿ × 2ⁿ Hadamard matrix may be constructed, following Sylvester construction: ${H}_{{2}^{n} \times {2}^{n}} = \left[\begin{matrix}{H}_{{2}^{n - 1} \times {2}^{n - 1}} & {H}_{{2}^{n - 1} \times {2}^{n - 1}} \\ {H}_{{2}^{n - 1} \times {2}^{n - 1}} & - {H}_{{2}^{n - 1} \times {2}^{n - 1}}\end{matrix}\right] .$

This Hadamard matrix gives the signs applied to the individual measurement signals coming from a matrix row, wherein a +1 term corresponds to a + sign, and a -1 term corresponding to a - sign. The column number indicates a sensor number, and the row number a time step number. The number of measurement time steps is equal to P = 2ⁿ.

Considering a given row in the matrix, the different signals generated by the different sensors 110 coupled to the waveguide 120 can be described as the vector **s_{q}** for the q^{th} row of the sensor matrix.

By applying the sign combinations through the Hadamard matrix, that is, **A** = **H_{2ⁿ×2n},** where P = 2ⁿ, we get a signal vector **s_{PD}** for the sensors associated with a photodiode PD, wherein the measurement signals are given as **s**_{**meas** PD} = **H_{2ⁿ×2ⁿ}s_{q},** Each row in **s**_{**meas** PD} corresponds to a measurement signal detected by the photodiode at a different timestep. Each term of **s_{meas PD}** is constituted by a linear combination of the individual measurement signals from the sensors s₁, s₂, .... The individual measurement signals are identical between the time intervals t₁, t₂ ... t_{P} as the same photoacoustic experiments are repeated while flipping the applied signs (that is, changing the mode flanks used for the different sensors).

To recover, that is, to demultiplex the individual measurement signals by each sensor, postprocessing may be performed by multiplying **s_{meas PD}** by ${H}_{{2}^{n} \times {2}^{n}}^{- 1}$. This is possible because the proposed **H**_{2ⁿ×2ⁿ} Hadamard is invertible. Hence, this scheme allows multiplexing and demultiplexing the signals coming from different sensors 110 in the group of sensors 112 coupled to the same waveguide 120. As before, the same multiplexing matrix can be applied in parallel to all the rows in the 2D opto-mechanical sensor matrix, as each row is readout by a different photodiode.

In addition, by using this scheme, an SNR expression for the individual measurement signal measured by each sensor is provided as: ${SNR}_{i , j} = \frac{{s}_{i , j}}{\sqrt{{σ}_{TMN}^{2} + \frac{1}{P} {σ}_{PD}^{2}}} .$

It should be noted that for a P × P matrix, by implementing such readout procedure in parallel for all the rows, P time steps are necessary to reach this SNR for each sensor 110 for the full matrix. The noise contribution coming from the photodiode has now been reduced for each sensor by a factor 1/P below the denominator square root with respect to the multiplexing schemes cases discussed in relation to Fig. 5a above.

Hence the SNR has been improved by the multiplexing/demultiplexing scheme itself by reducing the contribution of the noise from the photodiode to the total noise.

The previous multiplexing scheme gives the optimized improvement of SNR as it relies on the implementation of Hadamard matrices. However, practical implementation of the multiplexing scheme can be limited by the fact that it may not always be possible to use a factor -1 in linear combinations of the individual measurements. Rather, if shape of the optical mode is dominated by the thermo-optic effect as discussed above in relation to Fig. 2, the right flank of the optical resonance is not accessible, preventing to implement -1 terms necessary for Hadamard multiplexing.

According to an alternative approach, the sensor device 100 may be configured to control the relations between the interrogation wavelengths and the resonance wavelengths of the sensors 110 for detecting measurement signals representing a matrix derived from the Hadamard matrix of the linear combinations of the individual measurements. An example of such a matrix will be described below, referring to the matrix as an S-matrix.

The S-matrix may be used instead of the Hadamard matrix, as it is done in multiple multiplexing schemes where implementing readout of signals with factors +1 and -1 is experimentally challenging. The S-matrix still provides SNR improvement (potentially by a factor P/4 on the photodiode noise variance contribution to the total measurement noise, where P is the number of measurements in a series performed by each photodiode).

To this end, a (2ⁿ - 1) × (2ⁿ - 1) matrix is considered. In other words, in the example below, the number of sensors 110 in a row is considered to be P = 2ⁿ - 1.

An **S**_{(2ⁿ-1)×(2ⁿ-1)} S-matrix can be calculated from an **H_{2ⁿ×2ⁿ}** Hadamard matrix obtained by Sylvester construction. To this end, the first column and the first row of the **H_{2ⁿ×2n}** matrix are removed (which comprise only +1 terms). Then, the signs of the ±1 terms in the obtained matrix are flipped, and the -1 terms are replaced by 0 terms. The obtained matrix is the **S**_{(2ⁿ-1)×(2ⁿ-1)} matrix. An example of this is shown below, for n = 2.

In this case, the Hadamard matrix is: ${H}_{4 \times 4} = \left[\begin{matrix}1 & 1 & 1 & 1 \\ 1 & - 1 & 1 & - 1 \\ 1 & 1 & - 1 & - 1 \\ 1 & - 1 & - 1 & 1\end{matrix}\right] .$

The above-described method for arriving at an **S**_{3×3} matrix is applied, giving the following **S**_{3×3} matrix: ${S}_{3 \times 3} = \left[\begin{matrix}1 & 0 & 1 \\ 0 & 1 & 1 \\ 1 & 1 & 0\end{matrix}\right] .$

A practical advantage of an **S**_{(2ⁿ-1)×(2ⁿ-1)} matrix is that it comprises only +1 and 0 terms. The +1 terms are implemented by aligning the readout laser on the left flank of the resonance curve from the interrogated OMUS sensor. The 0 term can be implemented by turning off or by moving the laser providing the interrogation wavelength out of the optical resonance.

It should be noted that since in an **H_{2ⁿ×2 ⁿ}** Hadamard matrix two rows are orthogonal, and since the first row comprises only +1 terms, this means that any other row comprises the same number 2n/2 = 2ⁿ⁻¹ of +1 and -1 terms. Hence, the corresponding S-matrix will comprise 2ⁿ⁻¹ - 1 = (P - 1)/2 of 0 terms and 2ⁿ⁻¹ - 1 = (P + 1)/2 of +1 terms in each row. Therefore, in case tunable lasers or tunable sensors are used for implementing such scheme, the number of lasers needed for an **S**_{P×P} matrix corresponds to (P + 1)/2 lasers. If the sensor device 100 comprises a large number of sensors 110, this implies that a number of lasers needed is approximately equal to half the number of sensors in a row.

In addition, by using this scheme, an SNR expression for the individual measurement signal measured by each sensor is provided as: ${SNR}_{A = {S}_{P \times P}} = \frac{{s}_{i , j}}{\sqrt{\frac{2 P}{P + 1} {σ}_{TMN}^{2} + \frac{4 P}{{\left(P+1\right)}^{2}} {σ}_{PD}^{2}}} .$

In case P >> 1, the SNR expression may be provided as: ${SNR}_{A = {S}_{P \times P}} = \frac{{s}_{i , j}}{\sqrt{2 {σ}_{TMN}^{2} + \frac{4}{P} {σ}_{PD}^{2}}} .$

Each of the SNR expressions above is provided for P measurement time steps.

The noise coming from the photodiode has now been reduced for each sensor by a factor 4/P below the denominator square root with respect to the multiplexing schemes where only one sensor is readout at the time per photodiode, as discussed in relation to Fig. 5a above.

It should be noted however that the TMN variance contribution to the total measurement noise has been increased by a factor 2.

Hence, the S-matrix scheme is particularly interesting to implement when the noise contributions of the opto-mechanical sensors 110 is dominated by the noise coming from the photodiode, which can be the case for example for opto-mechanical systems presenting a low optomechanical transduction, for devices operated with a wide mechanical bandwidth measurement range, or at low temperatures.

In addition, the TMN contribution is multiplied by a factor of 2, no matter the number of sensors in the 2D matrix. This implies that SNR levels and NEP levels provided may in a simple manner be matched to the SNR and the NEP levels due to TMN that are provided for one sensor readout at the time per photodiode. In fact, by just averaging twice, the SNR and the NEP levels may match the SNR and the NEP levels due to TMN that are provided for one sensor readout at the time *per* photodiode.

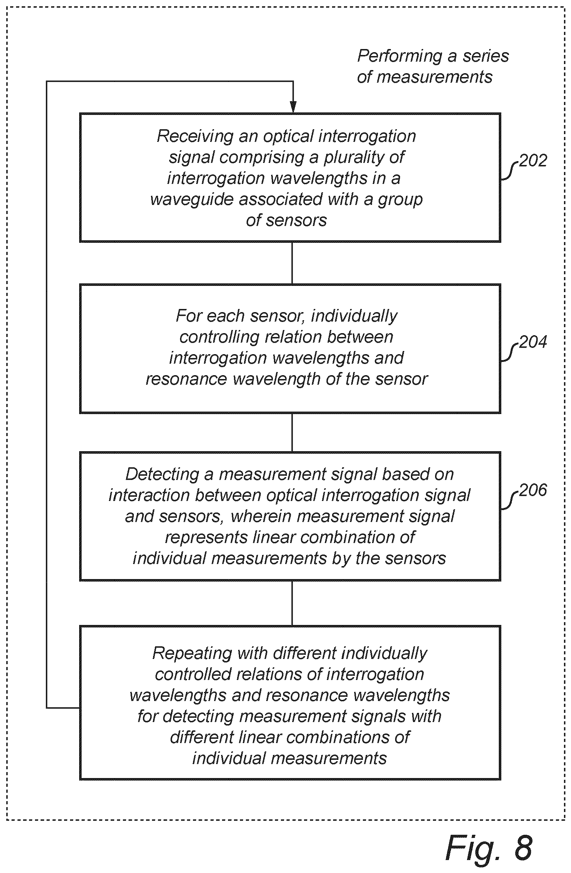

Referring now to Fig. 8, a method for acquiring information of a measurand will be discussed.

The method comprises performing a series of measurements of the measurand by a plurality of sensors. Each sensor comprises an optical resonance element, wherein an optical characteristic of the optical resonance element is configured to be affected by the measurand to be measured by the sensor.

Thus, multiple measurements in a series are performed. The method comprises performing a following procedure for each measurement in the series of measurements.

The method comprises receiving 202 an optical interrogation signal comprising a plurality of interrogation wavelengths in a waveguide associated with a group of sensors among the plurality of sensors.

The method further comprises for each sensor in the group of sensors, individually controlling 204 a relation between the plurality of interrogation wavelengths and the resonance wavelength of the sensor. The optical interrogation signal will interact with each of the sensors in the group of sensors. The interaction is dependent on the relation between an interrogation wavelength and the resonance wavelength of the sensor with which the interrogation wavelength is associated. The interaction will transfer an effect of the measurand on the optical resonance element onto a signal component at the interrogation wavelength associated with the sensor.

The method further comprises detecting 206 a measurement signal based on an intensity of an optical readout signal, which is based on the interaction of the optical interrogation signal with each of the sensors in the group of sensors. The measurement signal thus represents a linear combination of individual measurements of the measurand by the sensors in the group of sensors based on the controlling of the relation between the plurality of interrogation wavelengths and the resonance wavelengths of the sensors.

The method further comprises repeating the procedure of receiving 202, controlling 204, and detecting 206 for performing the plurality of measurements in the series of measurements. When repeating the procedure, the controlling 204 of the relation between the plurality of interrogation wavelengths and the resonance wavelength of each sensor provides different settings of the relations for different measurements in the series. The measurement signals in the series of measurements signals thus represent different linear combinations of the individual measurements of the measurand by the sensors in the group of sensors.

The series of measurement signals may be used for extracting the individual measurements of the measurand by the sensors. The individual measurements are provided with a high SNR.

In the above the inventive concept has mainly been described with reference to a limited number of examples. However, as is readily appreciated by a person skilled in the art, other examples than the ones disclosed above are equally possible within the scope of the inventive concept, as defined by the appended claims.

## Claims

1. A sensor device (100) comprising:
a plurality of sensors (110), wherein each sensor (110) comprises an optical resonance element (114), wherein an optical characteristic of the optical resonance element (114) is configured to be affected by a measurand to be measured by the sensor (110); and
a waveguide (120) associated with a group of sensors (112) among the plurality of sensors, wherein the waveguide (120) is configured to receive an optical interrogation signal comprising a plurality of separate interrogation wavelengths;
wherein the sensor device (100) is configured to, for each sensor (110), individually control a relation between the plurality of separate interrogation wavelengths and a resonance wavelength of optical resonance of the sensor (110);
wherein the sensor device (100) further comprises a detector (140) configured to receive an optical readout signal, wherein the optical readout signal is based on interaction of the optical interrogation signal with each of the sensors in the group of sensors (112), wherein the detector (140) is configured to detect a measurement signal based on an intensity of the optical readout signal, wherein the measurement signal represents a linear combination of individual measurements of the measurand by the sensors in the group of sensors (112);
wherein the sensor device (100) is configured to perform a series of measurements of the measurand, wherein the sensor device (100) is configured to provide different settings of the relations between the plurality of separate interrogation wavelengths and the resonance wavelengths of the sensors (110) for different measurements in the series, wherein the detector (140) is configured to detect a series of measurement signals for the series of measurements, wherein the measurement signals in the series of measurements signals represent different linear combinations of the individual measurements of the measurand by the sensors in the group of sensors (112).

2. The sensor device according to claim 1, wherein the detector (140) is a photodiode configured to detect intensity of the received optical readout signal.

3. The sensor device according to claim 1 or 2, wherein the waveguide (120) forms part of a plurality of waveguides, wherein each waveguide (120) is associated with a unique group of sensors (112), wherein a group of waveguides (120) among the plurality of waveguides are configured to simultaneously receive the optical interrogation signal for simultaneous readout of measurements from the groups of sensors (112) associated with the group of waveguides (120).

4. The sensor device according to claim 3, wherein the detector (140) forms part of a plurality of detectors, wherein each detector (140) is associated with a unique waveguide (120) for receiving the optical readout signal.

5. The sensor device according to claim 3 or 4, further comprising a splitter (150) configured to receive the optical interrogation signal and configured to split the optical interrogation signal to the group of waveguides.

6. The sensor device according to any one of the preceding claims, wherein the sensor device (100) further comprising a plurality of control elements (130), wherein each sensor (110) in the plurality of sensors is associated with a control element (130) of the plurality of control elements for individually controlling the relation between the plurality of separate interrogation wavelengths and the resonance wavelength of optical resonance of the sensor (110), wherein each control element (130) is individually controllable and configured to control the resonance wavelength of optical resonance of the sensor (110) associated with the control element (130) for tuning the resonance wavelength of the sensor (130), and wherein the plurality of control elements are configured to provide different settings of the resonance wavelengths of the sensors (110) for the different measurements in the series.

7. The sensor device according to claim 6, wherein each control element (130) is configured to tune the resonance wavelength of the sensor (110) associated with the control element (130) for aligning the resonance wavelength with a unique interrogation wavelength of the plurality of separate interrogation wavelengths.

8. The sensor device according to claim 6 or 7, wherein each control element (130) is configured to tune the resonance wavelength of the sensor (110) associated with the control element (130) for aligning the resonance wavelength with an interrogation wavelength of the plurality of separate interrogation wavelengths by the interrogation wavelength being arranged at a flank of a resonance curve of the resonance wavelength.

9. The sensor device according to any one of claims 6-8, wherein the control element (130) is configured to tune the resonance wavelength of the sensor (110) associated with the control element (130) for providing a positive sign or a negative sign of the measurement of the measurand by the sensor (110) in the linear combination of the measurement signal, wherein the control element (130) is configured tune the resonance wavelength of the sensor (110) associated with the control element (130) for providing the positive sign by the interrogation wavelength being arranged at a flank of negative slope of the resonance curve of the resonance wavelength, and wherein the control element (130) is configured tune the resonance wavelength of the sensor (110) associated with the control element (130) for providing the negative sign by the interrogation wavelength being arranged at a flank of positive slope of the resonance curve of the resonance wavelength.

10. The sensor device according to any one of the preceding claims, wherein the sensor device (100) is configured to, for each sensor (110), control the relation between the plurality of separate interrogation wavelengths and the resonance wavelength of the sensor (110) for providing a factor of the measurement of the measurand by the sensor (110) in the linear combination of the measurement signal, wherein the sensor device (100) is configured to control the relation for selecting the factor from a group of values comprising two or more of: +1, 0, and -1.

11. The sensor device according to any one of the preceding claims, wherein the sensor device (100) is configured to perform the series of measurements of the measurand for detecting measurement signals representing a Hadamard matrix of the linear combinations of the individual measurements of the measurand by the sensors (110) in the group of sensors (112).

12. The sensor device according to any one of claims 1-10, wherein the sensor device (100) is configured to perform the series of measurements of the measurand for detecting measurement signals representing a matrix derived from a Hadamard matrix of the linear combinations of the individual measurements of the measurand by the sensors (110) in the group of sensors (112).

13. The sensor device according to any one of the preceding claims, further comprising a controller configured to provide control signals for controlling settings of the relations between the plurality of separate interrogation wavelengths and the resonance wavelengths of the sensors (110) for the series of measurements.

14. An opto-mechanical sensor device comprising:
the sensor device (100) according to any one of the preceding claims, wherein each sensor (110) of the plurality of sensors comprises an opto-mechanical element (116) for receiving the measurand, and wherein the opto-mechanical element (116) is configured to be affected by the received measurand so as to change the optical characteristic of the optical resonance element (114).

15. A method for acquiring information of a measurand, said method comprising:
performing a series of measurements of the measurand by a plurality of sensors, wherein each sensor comprises an optical resonance element, wherein an optical characteristic of the optical resonance element is configured to be affected by the measurand to be measured by the sensor; and
for each measurement in the series of measurements:
receiving (202) an optical interrogation signal comprising a plurality of interrogation wavelengths in a waveguide associated with a group of sensors among the plurality of sensors;
for each sensor in the group of sensors, individually controlling (204) a relation between the plurality of interrogation wavelengths and the resonance wavelength of the sensor; and
detecting (206) a measurement signal based on an intensity of an optical readout signal, which is based on interaction of the optical interrogation signal with each of the sensors in the group of sensors, wherein the measurement signal represents a linear combination of individual measurements of the measurand by the sensors in the group of sensors,
wherein the controlling of the relation between the plurality of interrogation wavelengths and the resonance wavelength of each sensor provides different settings of the relations for different measurements in the series, wherein the measurement signals in the series of measurements signals represent different linear combinations of the individual measurements of the measurand by the sensors in the group of sensors.
